# EUROPEAN PATENT SPECIFICATION

(11) **EP 1 425 403 B1**
(45) Date of publication and mention of the grant of the patent: **31.12.2008**
(21) Application number: 02758603.1
(22) Date of filing: 12.09.2002
(51) Int. Cl.: C12N 15/86, A61K 48/00, A61P 25/04

(54) **LENTIVIRAL VECTORS FOR THE TREATMENT OF PAIN**
LENTIVIRALE VEKTOREN ZUR BEHANDLUNG VON SCHMERZEN
VECTEURS LENTIVIRAUX DANS LE TRAITEMENT DE LA DOULEUR

(30) Priority: 14.09.2001 GB 0122237; 08.05.2002 GB 0210575
(43) Date of publication of application: 09.06.2004
(73) Proprietor: Oxford Biomedica (UK) Limited, Oxford 0X4 4GA (GB)
(72) Inventor: BARBER, Rob Oxford BioMedica (UK) Limited, Oxford OX4 4GA (GB); AZZOUZ, Mimoun Oxford BioMedica (UK) Limited, Oxford OX4 4GA (GB); MAZARAKIS, Nick Oxford BioMedica (UK) Limited, Oxford OX4 4GA (GB); KINGSMAN, Susan Oxford BioMedica (UK) Limited, Oxford OX4 4GA (GB)
(74) Representative: Holliday, Louise Caroline
(86) International application number: PCT/GB2002/004169
(87) International publication number: WO 2003/025188

(56) References cited:
- WO-A-00/18903
- WO-A-02/36170
- WO-A-99/61639
- DATABASE BIOSIS [Online] BIOSCIENCES INFORMATION SERVICE, PHILADELPHIA, PA, US; April 2001 (2001-04) GOSS J R ET AL: "Antinociceptive effect of a genomic herpes simplex virus-based vector expressing human proenkephalin in rat dorsal root ganglion." Database accession no. PREV200100220095 XP002231111 & GENE THERAPY, vol. 8, no. 7, April 2001 (2001-04), pages 551-556, ISSN: 0969-7128
- MITROPHANOUS K A ET AL: "STABLE GENE TRANSFER TO THE NERVOUS SYSTEM USING A NON-PRIMATE LENTIVIRAL VECTOR" GENE THERAPY, MACMILLAN PRESS LTD., BASINGSTOKE, GB, vol. 6, no. 11, 1999, pages 1808-1818, XP000914884 ISSN: 0969-7128
- DATABASE BIOSIS [Online] BIOSCIENCES INFORMATION SERVICE, PHILADELPHIA, PA, US; 1991 TSIANG H ET AL: "RABIES VIRUS INFECTION AND TRANSPORT IN HUMAN SENSORY DORSAL ROOT GANGLIA NEURONS" Database accession no. PREV199192030308 XP002231112 & JOURNAL OF GENERAL VIROLOGY, vol. 72, no. 5, 1991, pages 1191-1194, ISSN: 0022-1317
- DATABASE BIOSIS [Online] BIOSCIENCES INFORMATION SERVICE, PHILADELPHIA, PA, US; 2000 OUDEGA M ET AL: "Amelioration of chronic neuropathic pain by adeno-associated viral (AAV) vector-mediated overexpression of BDNF in the rat spinal cord." Database accession no. PREV200100109054 XP002231113 & SOCIETY FOR NEUROSCIENCE ABSTRACTS, vol. 26, no. 1-2, 2000, pages Abstract No.-633.11, 30th Annual Meeting of the Society of Neuroscience;New Orleans, LA, USA; November 04-09, 2000 ISSN: 0190-5295
- MAZARAKIS N D ET AL: "RABIES VIRUS GLYCOPROTEIN PSEUDOTYPING OF LENTIVIRAL VECTORS ENABLES RETROGRADE AXONAL TRANSPORT AND ACCESS TO THE NERVOUS SYSTEM AFTER PERIPHERAL DELIVERY" HUMAN MOLECULAR GENETICS, OXFORD UNIVERSITY PRESS, SURREY, GB, vol. 10, no. 19, 15 September 2001 (2001-09-15), pages 2109-2121, XP001058914 ISSN: 0964-6906
- DATABASE BIOSIS [Online] BIOSCIENCES INFORMATION SERVICE, PHILADELPHIA, PA, US; 2001 AZZOUZ M ET AL: "Gene transfer to the nervous system using Equine Infectious Anaemia Virus based lentiviral vectors." Database accession no. PREV200100497474 XP002231114 & SOCIETY FOR NEUROSCIENCE ABSTRACTS, vol. 27, no. 1, 2001, page 526 31st Annual Meeting of the Society for Neuroscience;San Diego, California, USA; November 10-15, 2001 ISSN: 0190-5295

## Description

### FIELD OF THE INVENTION

The present invention relates to the use of a vector system. In particular, the present invention relates to the use of a vector system capable of delivering an entity of interest ("EOI") - such as a nucleotide sequence of interest ("NOI") - to a Dorsal Root Ganglion (DRG) of a subject.

The vector system is used in the manufacture of a medicament for use in a method for treating and/or preventing pain in a subject

The present invention also relates to the use of such a vector system in a screening method for identifying and/or validating an EOI useful in pain relief. In this method, the EOI is introduced to a target cell *in situ* within the DRG, *in vivo* within a non-human subject.

### BACKGROUND TO THE INVENTION

Production of an electrical signal in a cell is dependent on two basic features of the plasma membrane of excitable cells: the existence of a resting membrane potential and the presence of specific ion channels. The resting membrane potential is an electrical voltage difference across the membrane. The ion channels in the membrane open (or close) in response to specific stimuli, allowing specific ions to diffuse across the plasma membrane down their electro-chemical gradient. The result is a flow of current, which can change the membrane potential of the cell.

There are many instances in which it is desirable to modulate the membrane potential of an excitable cell. For example, pain is transmitted from the periphery into the central nervous system via a sensory nerve impulse. Modulation of the excitability of the sensory neuron responsible provides an approach to control pain.

The conventional method for treating pain is the administration of drugs such as anaesthetics, capsaisin, NSAIDs, opioids, NMDA antagonists and dorsal horn inhibitors. A recent survey showed that at least 40% of patients do not get adequate relief using such drugs.

The abstract Biosis PREV200100220095 discloses HSV mediated expression of enkephalin producing an antinociceptive effect, vector being administered by footpad delivery.

For the treatment of chronic pain, long term treatment is necessary, entailing indefinite frequent repeat doses of the drug, which is inconvenient and expensive.

The systemic administration of drugs needed only in small areas of the body is also associated with many side effects.

It is therefore desirable to develop a new approach to the treatment of pain, in particular chronic intransient pain, which has greater efficacy, specificity and possibly reduces the frequency and/or number of repeat treatments.

### SUMMARY OF ASPECTS OF THE PRESENT INVENTION

In a first aspect, the present invention relates to the use of a vector system in the manufacture of a medicament for use in a method for treating and/or preventing pain in a subject. The method involves administering a vector system that is capable of delivering an entity of interest ("EOI") to a DRG.

Cell bodies of sensory neurons are found in the DRG. The vector system may thus be capable of delivering the EOI to a sensory neuron.

In a first preferred aspect the EOI is capable of modulating the cellular excitability of a target cell, for example a sensory neuron.

In a second preferred aspect the EOI is capable of modulating the expression or activity of a receptor, such as an opioid receptor or an NMDA receptor.

In a third preferred aspect the EOI is capable of encoding a neurotrophic factor, such as glial cell-derived neurotrophic factor (GDNF)

The vector system is a lentiviral vector system.

The present invention provides the use of a lentiviral vector system pseudotyped with at least part of a rabies G protein or a mutant, variant, homologue or fragment thereof in the manufacture of a medicament for use in a method for treating and/or preventing pain in a subject, which method comprises the step of administration of the lentiviral vector system such that it delivers an Entity of Interest (EOI) to a Dorsal Root Ganglion (DRG) of the subject, wherein
(i) the vector system or part thereof travels from the administration site to the DRG by retrograde transport; and
(ii) said pseudotyping entity has the activity of enabling the vector system to travel by retrograde transport to the DRG.

The vector system is administered to a site which is distant to the DRG. The vector system (or part thereof) then travels to the DRG by retrograde transport.

The vector system is pseudotyped with at least a part of a rabies G protein (glycoprotein) or a mutant, variant, homologue or fragment thereof which causes the system to travel by retrograde transport.

Administration to a site which is distant to the DRG is advantageous because such a site may be more accessible than the DRG. Also, by using retrograde transport is it possible to deliver the EOI to certain cells or groups of cells. For example, where the vector system is administered peripherally at the site of pain, the vector system (or part thereof) will travel to the DRG by retrograde transport and deliver the EOI to cells which are directly involved in sensing the pain.

The vector system may be used for delivering an EOI to the spinal cord, which comprises the following steps:
(i) delivery of an EOI to the cell body of a sensory neuron within the DRG;
(ii) optional modification of the EOI; and
(iii) delivery of the optionally modified EOI from the cell body of the sensory neuron to the spinal cord via the central branch of the sensory neuron.

The present invention also provides a screening method for identification and/or validation of an EOI useful in the prevention and/or treatment of pain which comprises the following steps
(i) administration of a lentiviral vector system as defined herein to a non-human subject such that it delivers a test EOI to a target cell in the subject by the method according to the first aspect of the invention , wherein the target cell is in *situ* within the DRG;
(ii) analysis of effect of the EOI on the target cell; and
(iii) selection of an EOI with therapeutic potential.

As used herein, the term "treatment" includes curative effects, alleviation effects, and prophylactic effects.

Analysis of the effect of the EOI on the target cell may involve monitoring EOI-induced modulation of the transcriptome and/or proteotome of the target cell. In this way novel genes may be identified as a result of their capacity to modulate the transcriptome/proteotome or as a result of EOI-induced modulation of their transcription/translation.

The method may involve analysis of pain in the subject.

The method may involve *in vitro* screening for an EOI with therapeutic potential, followed by *in vivo* verification of its therapeutic effect, by i) delivery of the selected EOI to the DRG of the subject and ii) analysis of pain in the subject.

### DETAILED DESCRIPTION OF THE INVENTION

The present invention relates to a new use of a vector system.

As used herein the term "vector system" includes any vector that is capable of infecting or transducing or transforming or modifying a recipient cell with an EOI.

The vector system is a lentivial vector system.

### RETROVIRUSES

The concept of using viral vectors for gene therapy is well known (Verma and Somia (1997) Nature 389:239-242).

There are many retroviruses. For the present application, the term "retrovirus" includes: murine leukemia virus (MLV), human immunodeficiency virus (HIV), equine infectious anaemia virus (EIAV), mouse mammary tumour virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV) and all other retroviridiae including lentiviruses.

A detailed list of retroviruses may be found in Coffin et al ("Retroviruses" 1997 Cold Spring Harbour Laboratory Press Eds: JM Coffin, SM Hughes, HE Varmus pp 758-763).

The retroviral vector system used in the present invention is derivable from a lentivirus. Lentiviruses also belong to the retrovirus family, but they can infect both dividing and non-dividing cells (Lewis et al (1992). EMBO J. 3053-3058).

The lentivirus group can be split into "primate" and "non-primate". Examples of primate lentiviruses include the human immunodeficiency virus (HIV), the causative agent of human acquired immunodeficiency syndrome (AIDS), and the simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV). In a preferred embodiment, the retroviral vector system is derivable from EIAV.

Details on the genomic structure of some lentiviruses may be found in the art. By way of example, details on HIV and EIAV may be found from the NCBI Genbank database (i.e. Genome Accession Nos. AF033819 and AF033820 respectively).

Details of HIV variants may also be found at http://hiv-web.lanl.gov. Details of EIAV variants may be found through http://www.ncbi.nlm.nih.gov.

During the process of infection, a retrovirus initially attaches to a specific cell surface receptor. On entry into the susceptible host cell, the retroviral RNA genome is then copied to DNA by the virally encoded reverse transcriptase which is carried inside the parent virus. This DNA is transported to the host cell nucleus where it subsequently integrates into the host genome. At this stage, it is typically referred to as the provirus. The provirus is stable in the host chromosome during cell division and is transcribed like other cellular genes. The provirus encodes the proteins and other factors required to make more virus, which can leave the cell by a process sometimes called "budding".

Each retroviral genome comprises genes called *gag*, *pol* and *env* which code for virion proteins and enzymes. These genes are flanked at both ends by regions called long terminal repeats (LTRs), The LTRs are responsible for proviral integration, and transcription. They also serve as enhancer-promoter sequences. In other words, the LTRs can control the expression of the viral genes. Encapsidation of the retroviral RNAs occurs by virtue of a psi sequence located at the 5' end of the viral genome.

The LTRs themselves are identical sequences that can be divided into three elements, which are called U3, R and U5. U3 is derived from the sequence unique to the 3' end of the RNA. R is derived from a sequence repeated at both ends of the RNA and U5 is derived from the sequence unique to the 5'end of the RNA. The sizes of the three elements can vary considerably among different retroviruses.

For the viral genome, the site of transcription initiation is at the boundary between U3 and R in one LTR and the site of poly (A) addition (termination) is at the boundary between R and U5 in the other LTR. U3 contains most of the transcriptional control elements of the provirus, which include the promoter and multiple enhancer sequences responsive to cellular and in some cases, viral transcriptional activator proteins. Some retroviruses have any one or more of the following genes that code for proteins that are involved in the regulation of gene expression: *tat, rev, tax* and *rex.*

With regard to the structural genes *gag, pol* and *env* themselves, gag encodes the internal structural protein of the virus. Gag protein is proteolytically processed into the mature proteins MA (matrix), CA (capsid) and NC (nucleocapsid). The *pol* gene encodes the reverse transcriptase (RT), which contains DNA polymerase, associated RNase H and integrase (IN), which mediate replication of the genome. The env gene encodes the surface (SU) glycoprotein and the transmembrane (TM) protein of the virion, which form a complex that interacts specifically with cellular receptor proteins. This interaction leads ultimately to infection by fusion of the viral membrane with the cell membrane.

Retroviruses may also contain "additional" genes which code for proteins other than gag, pol and env. Examples of additional genes include in HIV, one or more of *vif, vpr, vpx, vpu, tat, rev* and *nef.* EIAV *tat, rev* and S2.

Proteins encoded by additional genes serve various functions, some of which may be duplicative of a function provided by a cellular protein. In EIAV, for example, *tat* acts as a transcriptional activator of the viral LTR. It binds to a stable, stem-loop RNA secondary structure referred to as TAR. Rev regulates and co-ordinates the expression of viral genes through rev-response elements (RRE). The mechanisms of action of these two proteins are thought to be broadly similar to the analogous mechanisms in the primate viruses. The function of S2 is unknown. In addition, an EIAV protein, Ttm, has been identified that is encoded by the first exon of *tat* spliced to the env coding sequence at the start of the transmembrane protein.

As used herein the term "vector system", also includes a vector particle capable of transducing a recipient cell with an NOI.

A vector particle includes the following components: a vector genome, which may contain one or more NOIs, a nucleocapsid encapsidating the nucleic acid, and a membrane surrounding the nucleocapsid.

The term "nucleocapsid" refers to at least the group specific viral core proteins (gag) and the viral polymerase (pol) of a retrovirus genome. These proteins encapsidate the packagable sequences and are themselves further surrounded by a membrane containing an envelope glycoprotein.

Once within the cell, the RNA genome from a retroviral vector particle is reverse transcribed into DNA and integrated into the DNA of the recipient cell.

The term "vector genome" refers to both to the RNA construct present in the retroviral vector particle and the integrated DNA construct. The term also embraces a separate or isolated DNA construct capable of encoding such an RNA genome. A retroviral or lentiviral genome should comprise at least one component part derivable from a retrovirus or a lentivirus. The term "derivable" is used in its normal sense as meaning a nucleotide sequence or a part thereof which need not necessarily be obtained from a virus such as a lentivirus but instead could be derived therefrom. By way of example, the sequence may be prepared synthetically or by use of recombinant DNA techniques. Preferably the genome comprises a *psi* region (or an analogous component which is capable of causing encapsidation).

The viral vector genome is preferably "replication defective" by which we mean that the genome does not comprise sufficient genetic information alone to enable independent replication to produce infectious viral particles within the recipient cell. In a preferred embodiment, the genome lacks a functional *env, gag* or pol gene. In a highly preferred embodiment the genome lacks *env*, *gag* and *pol* genes.

The viral vector genome may comprise some or all of the long terminal repeats (LTRs). Preferably the genome comprises at least part of the LTRs or an analogous sequence which is capable of mediating proviral integration, and transcription. The sequence may also comprise or act as an enhancer-promoter sequence.

It is known that the separate expression of the components required to produce a retroviral vector particle on separate DNA sequences cointroduced into the same cell will yield retroviral particles carrying defective retroviral genomes that carry therapeutic genes (e.g. Reviewed by Miller 1992). This cell is referred to as the producer cell (see below).

There are two common procedures for generating producer cells, In one, the sequences encoding retroviral Gag, Pol and Env proteins are introduced into the cell and stably integrated into the cell genome; a stable cell line is produced which is referred to as the packaging cell line. The packaging cell line produces the proteins required for packaging retroviral RNA but it cannot bring about encapsidation due to the lack of a *psi* region. However, when a vector genome (having a *psi* region) is introduced into the packaging cell line, the helper proteins can package the *psi-*positive recombinant vector RNA to produce the recombinant virus stock. This can be used to transduce the NOI into recipient cells. The recombinant virus whose genome lacks all genes required to make viral proteins can infect only once and cannot propagate. Hence, the NOI is introduced into the host cell genome without the generation of potentially harmful retrovirus. A summary of the available packaging lines is presented in "Retroviruses" (1997 Cold Spring Harbour Laboratory Press Eds: JM Coffin, SM Hughes, HE Varmus pp 449).

A packaging cell line comprising a viral vector genome may be used a vector system for use in the invention. For example, the packaging cell line may be transduced with a viral vector system comprising the genome or transfected with a plasmid carrying a DNA construct capable of encoding the RNA genome.

The second approach is to introduce the three different DNA sequences that are required to produce a retroviral vector particle i.e. the *env* coding sequences, the *gag-*pol coding sequence and the defective retroviral genome containing one or more NOIs into the cell at the same time by transient transfection and the procedure is referred to as transient triple transfection (Landau & Littman 1992; Pear et al 1993). The triple transfection procedure has been optimised (Soneoka et al 1995; Finer et al 1994). WO 94/29438 describes the production of producer cells *in vitro* using this multiple DNA transient transfection method. WO 97127310 describes a set of DNA sequences for creating retroviral producer cells either *in vivo* or *in vitro* for re-implantation.

The components of the viral system which are required to complement the vector genome may be present on one or more "producer plasmids" for transfecting into cells.

A kit for producing a retroviral vector for use in the invention may comprise
(i) a viral vector genome which is incapable of encoding one or more proteins which are required to produce a vector particles;
(ii) one or more producer plasmid(s) capable of encoding the protein which is not encoded by (i); and optionally
(iii) a cell suitable for conversion into a producer cell.

The viral vector genome may be incapable of encoding the proteins gag, pol and env. The kit may comprise one or more producer plasmids encoding env, gag and pol, for example, one producer plasmid encoding env and one encoding gag-pol. The the gag-pol sequence may be codon optimised for use in the particular producer cell (see below).

A producer cell expressing the vector genome and the producer plasmid(s) may be used to produce a retroviral vector system for use in the present invention.

Preferably the retroviral vector system used in the present invention is a self-inactivating (SIN) vector system.

By way of example, self-inactivating retroviral vector systems have been constructed by deleting the transcriptional enhancers or the enhancers and promoter in the U3 region of the 3' LTR. After a round of vector reverse transcription and integration, these changes are copied into both the 5' and the 3' LTRs producing a transcriptionally inactive provirus. However, any promoter(s) internal to the LTRs in such vectors will still be transcriptionally active. This strategy has been employed to eliminate effects of the enhancers and promoters in the viral LTRs on transcription from internally placed genes. Such effects include increased transcription or suppression of transcription. This strategy can also be used to eliminated downstream transcription from the 3' LTR into genomic DNA. This is of particular concern in human gene therapy where it may be important to prevent the adventitious activation of an endogenous oncogene.

A recombinase assisted mechanism may be used which facilitates the production of high titre regulated lentiviral vectors from producer cells.

As used herein, the term "recombinase assisted system" includes but is not limited to a system using the Cre recombinase / IoxP recognition sites of bacteriophage P1 or the site-specific FLP recombinase of *S*. *cerevisiae* which catalyses recombination events between 34 bp FLP recognition targets (FRTs).

The site-specific FLP recombinase of *S*. *cerevisiae* which catalyses recombination events between 34 bp FLP recognition targets (FRTs) has been configured into DNA constructs in order to generate high level producer cell lines using recombinase-assisted recombination events (Karreman et al (1996) NAR 24:1616-1624). A similar system has been developed using the Cre recombinase / IoXP recognition sites of bacteriophage P1 (see PCT/GB00/03837; Vanin et al (1997) J. Virol 71:7820-7826). This was configured into a lentiviral genome such that high titre lentiviral producer cell lines were generated.

By using producer/packaging cell lines, it is possible to propagate and isolate quantities of retroviral vector particles (e.g. to prepare suitable titres of the retroviral vector particles) for subsequent transduction of, for example, a site of interest (such as a DRG). Producer cell lines are usually better for large-scale production or vector particles.

Transient transfection has numerous advantages over the packaging cell method. In this regard, transient transfection avoids the longer time required to generate stable vector-producing cell lines and is used if the vector genome or retroviral packaging components are toxic to cells. If the vector genome encodes toxic genes or genes that interfere with the replication of the host cell, such as inhibitors of the cell cycle or genes that induce apoptosis, it may be difficult to generate stable vector-producing cell lines, but transient transfection can be used to produce the vector before the cells die. Also, cell lines have been developed using transient infection that produce vector titre levels that are comparable to the levels obtained from stable vector-producing cell lines (Pear et al 1993, PNAS 90:8392-8396).

Producer cells/packaging cells can be of any suitable cell type. Producer cells are generally mammalian cells but can be, for example, insect cells.

As used herein, the term "producer cell" or "vector producing cell" refers to a cell which contains all the elements necessary for production of retroviral vector particles.

Preferably the envelope protein sequences, and nucleocapsid sequences are all stably integrated in the producer and/or packaging cell. However, one or more of these sequences could also exist in episomal form and gene expression could occur from the episome.

As used herein, the term "packaging cell" refers to a cell which contains those elements necessary for production of infectious recombinant virus which are lacking in the RNA genome. Typically, such packaging cells contain one or more producer plasmids which are capable of expressing viral structural proteins (such as *gag-pol* and *env,* which may be codon optimised) but they do not contain a packaging signal.

The term "packaging signal" which is referred to interchangeable as "packaging sequence" or "*psi*" is used in reference to the non-coding, cis-acting sequence required for encapsidation of retroviral RNA strands during viral particle formation. In HIV-1, this sequence has been mapped to loci extending from upstream of the major splice donor site (SD) to at least the *gag* start codon.

Packaging cell lines may be readily prepared (see also WO 92/05266), and utilised to create producer cell lines for the production of retroviral vector particles. As already mentioned, a summary of the available packaging lines is presented in "Retroviruses" (as above).

Also as discussed above, simple packaging cell lines, comprising a provirus in which the packaging signal has been deleted, have been found to lead to the rapid production of undesirable replication competent viruses through recombination. In order to improve safety, second generation cell lines have been produced wherein the 3'LTR of the provirus is deleted. In such cells, two recombinations would be necessary to produce a wild type virus. A further improvement involves the introduction of the *gag-pol* genes and the *env* gene on separate constructs so-called third generation packaging cell lines. These constructs are introduced sequentially to prevent recombination during transfection.

In these split-construct, third generation cell lines, a further reduction in recombination may be achieved by changing the codons. This technique, based on the redundancy of the genetic code, aims to reduce homology between the separate constructs, for example between the regions of overlap in the gag-pol and env open reading frames.

The packaging cell lines are useful for providing the gene products necessary to encapsidate and provide a membrane protein for a high titre vector particle production. The packaging cell may be a cell cultured *in vitro* such as a tissue culture cell line. Suitable cell lines include but are not limited to mammalian cells such as murine fibroblast derived cell lines or human cell lines. Preferably the packaging cell line is a human cell line, such as for example: HEK293, 293-T, TE671, HT1080.

Alternatively, the packaging cell may be a cell derived from the individual to be treated such as a monocyte, macrophage, blood cell or fibroblast The cell may be isolated from an individual and the packaging and vector components administered *ex vivo* followed by re-administration of the autologous packaging cells.

It is highly desirable to use high-titre virus preparations in both experimental and practical applications. Techniques for increasing viral titre include using a *psi* plus packaging signal as discussed above and concentration of viral stocks.

As used herein, the term "high titre" means an effective amount of a retroviral vector or particle which is capable of transducing a target site such as a cell.

As used herein, the term "effective amount" means an amount of a regulated retroviral or lentiviral vector or vector particle which is sufficient to induce expression of the NOIs at a target site.

A high-titre viral preparation for a producer/packaging cell is usually of the order of 10⁶ to 10⁷ t.u. per ml. (The titer is expressed in transducing units per ml (t.u./ml) as titred on a standard D17 cell line). For transduction in tissues such as the DRG, it is necessary to use very small volumes, so the viral preparation is concentrated by ultracentrifugation. The resulting preparation should have at least 10⁸ t.u./ml, preferably from 10⁸ to 10⁹ t.u./ml, more preferably at least 10⁹ t.u./ml.

The presence of a sequence termed the central polypurine tract (cPPT) may improve the efficiency of gene delivery to non-dividing cells (see WO 00/31200). This *cis-*acting element is located, for example, in the EIAV polymerase coding region element. Preferably the genome of the vector system used in the present invention comprises a cPPT sequence.

In addition, or in the alternative, the viral genome may comprise a post-translational regulatory element and/or a translational enhancer.

### MINIMAL SYSTEMS

It has been demonstrated that a primate lentivirus minimal system can be constructed which requires none of the HIV/SIV additional genes *vif*, *vpr*, *vpx*, *vpu*, *tat, rev* and *nef* for either vector production or for transduction of dividing and non-dividing cells. It has also been demonstrated that an EIAV minimal vector system can be constructed which does not require S2 for either vector production or for transduction of dividing and non-dividing cells. The deletion of additional genes is highly advantageous. Firstly, it permits vectors to be produced without the genes associated with disease in lentiviral (e.g. HIV) infections. In particular, *tat* is associated with disease. Secondly, the deletion of additional genes permits the vector to package more heterologous DNA. Thirdly, genes whose function is unknown, such as S2, may be omitted, thus reducing the risk of causing undesired effects. Examples of minimal lentiviral vectors are disclosed in WO-A-99/32646 and in WO-A-98/17815.

Thus, preferably, the delivery system used in the invention is devoid of at least *tat* and S2 (if it is an EIAV vector system), and possibly also *vif*, *vpr*, *vpx*, *vpu* and *nef*. More preferably, the systems of the present invention are also devoid of *rev*. Rev was previously thought to be essential in some retroviral genomes for efficient virus production. For example, in the case of HIV, it was thought that *rev* and RRE sequence should be included. However, it has been found that the requirement for *rev* and RRE can be reduced or eliminated by codon optimisation (see below) or by replacement with other functional equivalent systems such as the MPMV system. As expression of the codon optimised *gag-pol* is REV independent, RRE can be removed from the *gag-pol* expression cassette, thus removing any potential for recombination with any RRE contained on the vector genome.

In a preferred embodiment the viral genome of the first aspect of the invention lacks the Rev response element (RRE).

In a preferred embodiment, the system used in the present invention is based on a so-called "minimal" system in which some or all of the additional genes have be removed.

### CODON OPTIMISATION

Codon optimisation has previously been described in WO99/41397. Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available.

Many viruses, including HIV and other lentiviruses, use a large number of rare codons and by changing these to correspond to commonly used mammalian codons, increased expression of the packaging components in mammalian producer cells can be achieved. Codon usage tables are known in the art for mammalian cells, as well as for a variety of other organisms.

Codon optimisation has a number of other advantages. By virtue of alterations in their sequences, the nucleotide sequences encoding the packaging components of the viral particles required for assembly of viral particles in the producer cells/packaging cells have RNA instability sequences (INS) eliminated from them. At the same time, the amino acid sequence coding sequence for the packaging components is retained so that the viral components encoded by the sequences remain the same, or at least sufficiently similar that the function of the packaging components is not compromised. Codon optimisation also overcomes the Rev/RRE requirement for export, rendering optimised sequences Rev independent. Codon optimisation also reduces homologous recombination between different constructs within the vector system (for example between the regions of overlap in the gag-pol and env open reading frames). The overall effect of codon optimisation is therefore a notable increase in viral titre and improved safety.

In one embodiment only codons relating to INS are codon optimised. However, in a much more preferred and practical embodiment, the sequences are codon optimised in their entirety, with the exception of the sequence encompassing the frameshift site.

The *gag-pol* gene comprises two overlapping reading frames encoding the gag-pol proteins. The expression of both proteins depends on a frameshift during translation. This frameshift occurs as a result of ribosome "slippage" during translation. This slippage is thought to be caused at least in part by ribosome-stalling RNA secondary structures. Such secondary structures exist downstream of the frameshift site in the - *gag-pol* gene. For HIV, the region of overlap extends from nucleotide 1222 downstream of the beginning of *gag* (wherein nucleotide 1 is the A of the *gag* ATG) to the end of *gag* (nt 1503). Consequently, a 281 bp fragment spanning the frameshift site and the overlapping region of the two reading frames is preferably not codon optimised. Retaining this fragment will enable more efficient expression of the gag-pol proteins.

For EIAV the beginning of the overlap has been taken to be nt 1262 (where nucleotide 1 is the A of the *gag* ATG). The end of the overlap is at 1461 bp. In order to ensure that the frameshift site and the *gag-pol* overlap are preserved, the wild type sequence has been retained from nt 1156 to 1465.

Derivations from optimal codon usage may be made, for example, in order to accommodate convenient restriction sites, and conservative amino acid changes may be introduced into the gag-pol proteins.

In a highly preferred embodiment, codon optimisation was based on lightly expressed mammalian genes. The third and sometimes the second and third base may be changed.

Due to the degenerate nature of the Genetic Code, it will be appreciated that numerous *gag-pol* sequences can be achieved by a skilled worker. Also there are many retroviral variants described which can be used as a starting point for generating a codon optimised *gag-pol* sequence. Lentiviral genomes can be quite variable. For example there are many quasi-species of HIV-1 which are still functional. This is also the case for EIAV. These variants may be used to enhance particular parts of the transduction process. Examples of HIV-1 variants may be found at http://hiv-web.lanl.gov. Details of EIAV clones may be found at the NCBI database: http://www.ncbi.nlm.nih.gov.

The strategy for codon optimised *gag-pol* sequences can be used in relation to any retrovirus. This would apply to all lentiviruses, including EIAV, FIV, BIV, CAEV, VMR, SIV, HIV and HIV-2. In addition this method could be used to increase expression of genes from HTLV-1, HTLV 2, HFV, HSRV and human endogenous retroviruses (HERV), MLV and other retroviruses.

Codon optimisation can render *gag-pol* expression Rev independent. In order to enable the use of anti-*rev* or RRE factors in the retroviral vector, however, it would be necessary to render the viral vector generation system totally Rev/RRE independent. Thus, the genome also needs to be modified. This is achieved by optimising vector genome components. Advantageously, these modifications also lead to the production of a safer system absent of all additional proteins both in the producer and in the transduced cell.

As described above, the packaging components for a retroviral vector include expression products of *gag, pol* and *env* genes. In addition, efficient packaging depends on a short sequence of 4 stem loops followed by a partial sequence from *gag* and *env* (the "packaging signal"), Thus, inclusion of a deleted *gag* sequence in the retroviral vector genome (in addition to the full *gag* sequence on the packaging construct) will optimise vector titre. To date efficient packaging has been reported to require from 255 to 360 nucleotides of *gag* in vectors that still retain *env* sequences, or about 40 nucleotides of gag in a particular combination of splice donor mutation, gag and env deletions. It has surprisingly been found that a deletion of all but the N-terminal 360 or so nucleotides in *gag* leads to an increase in vector titre. Thus, preferably, the retroviral vector genome includes a gag sequence which comprises one or more deletions, more preferably the gag sequence comprises about 360 nucleotides derivable from the N-terminus.

### PSEUDOTYPING

In the design of retroviral vector systems it is desirable to engineer particles with different target cell specificities to the native virus, to enable the delivery of genetic material to an expanded or altered range of cell types. One manner in which to achieve this is by engineering the virus envelope protein to alter its specificity. Another approach is to introduce a heterologous envelope protein into the vector particle to replace or add to the native envelope protein of the virus.

The term pseudotyping means incorporating at least a part of, or substituting a part of, or replacing all of, an *env* gene of a viral genome with a heterologous *env* gene, for example an env gene from another virus. Pseudotyping is not a new phenomenon and examples may be found in WO 99/61639, WO-A-98/05759, WO-A-98/05754, WO-A-97/17457, WO-A-96/09400, WO-A-91/00047 and Mebatsion et al 1997 Cell 90, 841-847.

Pseudotyping can improve retroviral vector stability and transduction efficiency. A pseudotype of murine leukemia virus packaged with lymphocytic choriomeningitis virus (LCMV) has been described (Miletic et al (1999) J. Virol. 73:6114-6116) and shown to be stable during ultracentrifugation and capable of infecting several cell lines from different species.

The lentiviral vector system of the present invention is pseudotyped with at least part of a rabies G protein or a mutant variant, homolog or fragment thereof.

For pseudotyped vector systems, the heterologous env region may be encoded by a gene which is present on a producer plasmid. The producer plasmid may be present as part of a kit for the production of retroviral vector particles suitable for use in the invention.

The pseudotyping entity has the activity of enabling the vector system to travel by retrograde transport to the DRG.

The vector system may comprise a first nucleotides sequence coding for at least a part of an envelope protein; and one or more other nucleotide sequences derivable from a retrovirus that ensure transduction by the retroviral delivery system; wherein the first nucleotide sequence is heterologous with respect to at least one of the other nucleotide sequences; and wherein the first nucleotide sequence codes for at least a part of a rabies G protein or a mutant, variant, homologue or fragment thereof.

### RABIES G PROTEIN

For use in the present invention, the vector system is pseudotyped with at least a part of a rabies G protein or a mutant, variant, homologue or fragment thereof.

Teachings on the rabies G protein, as well as mutants thereof, may be found in WO 99/61639 and well as Rose et al., 1982 J. Virol. 43: 361,364, Hanham et a/., 1993 J. Virol.,67, 530-542, Tuffereau et al.,1998 J. Virol., 72, 108a-1091, Kucera et al., 1985 J. Virol 55, 158-162, Dietzschold et al., 1983 PNAS 80, 70-74, Seif et al., 1985 J.Virol., 53, 926-934, Coulon et al.,1998 J. Virol., 72, 273-278, Tuffereau et al.,1998 J. Virol., 72, 1085-10910, Burger et al., 1991 J.Gen. Virol. 72. 359-367, Gaudin et al 1995 J Virol 69, 5528-5534, Benmansour et al 1991 J Virol 65, 4198-4203, Luo et al 1998 Microbiol Immunol 42, 187-193, Coll 1997 Arch Virol 142, 2089-2097, Luo et a/ 1997 Virus Res 51, 35-41, Luo et al 1998 Microbiol Immunol 42, 187-193, Coll 1995 Arch Virol 140, 827-851, Tuchiya et al 1992 Virus Res 25, 1-13, Morimoto et al 1992 Virology 189, 203-216, Gaudin et al 1992 Virology 187, 627-632, Whitt et al 1991 Virology 185, 681-688, Dietzschold et al 1978 J Gen Virol 40, 131-139, Dietzschold et al 1978 Dev Biol Stand 40, 45-55, Dietzschold et al 1977 J Virol 23, 286-293, and Otvos et al 1994 Biochim Biophys Acta 1224, 68-76. A rabies G protein is also described in EP-A-0445625.

The use of rabies G protein provides vectors which, *in vivo,* preferentially transduce targeted cells which rabies virus preferentially infects. This includes in particular neuronal target cells *in vivo.* For a neuron-targeted vector, rabies G from a pathogenic strain of rabies such as ERA may be particularly effective. On the other hand rabies G protein confers a wider target cell range *in vitro* including nearly all mammalian and avian cell types tested (Seganti et al., 1990 Arch Virol. 34,155-163; Fields et al., 1996 Fields Virology, Third Edition, vol.2, Lippincott-Raven Publishers, Philadelphia, New York).

The tropism of the pseudotyped vector particles may be modified by the use of a mutant rabies G which is modified in the extracellular domain. Rabies G protein has the advantage of being mutatable to restrict target cell range. The uptake of rabies virus by target cells *in vivo* is thought to be mediated by the acetylcholine receptor (AchR) but there may be other receptors to which it binds *in vivo* (Hanham et a/., 1993 J. Virol.,67, 530-542; Tuffereau et al.,1998 J. Virol., 72, 1085-1091). It is thought that multiple receptors are used in the nervous system for viral entry, including NCAM (Thoulouze et al (1998) J. Virol 72(9):7181-90) and p75 Neurotrophin receptor (Tuffereau C et al (1998) Embo J 17(24) 7250-9).

The effects of mutations in antigenic site III of the rabies G protein on virus tropism have been investigated and this region is not thought to be involved in the binding of the virus to the acetylcholine receptor (Kucera et al., 1985 J. Virol 55, 158-162; Dietzschold et al., 1983 Proc Natl Acad Sci 80, 70-74; Seif et al., 1985 J.Virol., 53, 926-934; Coulon et al.,1998 J. Virol., 72, 273-278; Tuffereau et al., 1998 J. Virol., 72, 1085-10910). For example a mutation of the arginine at amino acid 333 in the mature protein to glutamine can be used to restrict viral entry to olfactory and peripheral neurons *in vivo* while reducing propagation to the central nervous system. These viruses were able to penetrate motor neurons and sensory neurons as efficiently as the wild type virus, yet transneuronal transfer did not occur (Coulon et al., 1989, J. Virol. 63, 3550-3554). Viruses in which amino acid 330 has been mutated are further attenuated, being usable to infect either motor neurons or sensory neurons after intra-muscular injection (Coulon et a/.,1998 J. Virol., 72, 273-278).

Alternatively or additionally, rabies G proteins from laboratory passaged strains of rabies may be used. These can be screened for alterations in tropism. Such strains include the hollowing:

| **Genbank accession number** | **Rabies Strain** |
|---|---|
| J02293 | ERA |
| U52947 | COSRV |
| U27214 | NY 516 |
| U27215 | NY771 |
| U27216 | FLA125 |
| U52946 | SHBRV |
| M32751 | HEP-Flury |
| U17064 | Mokola G |
| * | |

Request ID for a blast of the sequence is available under 1000214283 -16535-22519.

By way of example, the ERA strain is a pathogenic strain of rabies and the rabies G protein from this strain can be used for transduction of neuronal cells. The sequence of rabies G from the ERA strains is in the GenBank database (accession number J02293). This protein has a signal peptide of 19 amino acids and the mature protein begins at the lysine residue 20 amino acids from the translation initiation methionine. The HEP-Flury strain contains the mutation from arginine to glutamine at amino acid position 333 in the mature protein which correlates with reduced pathogenicity and which can be used to restrict the tropism of the viral envelope.

WO 99/61639 discloses the nucleic and amino acid sequences for a rabies virus strain ERA (Genbank locus RAVGPLS, accession M38452).

### MUTANTS, VARIANTS, HOMOLOGUES AND FRAGMENTS

The vector system used in the present invention is pseudotyped with at least part of a wild-type rabies G protein or a mutant, variant, homologue or fragment thereof.

The term "wild type" is used to mean a polypeptide having a primary amino acid sequence which is identical with the native protein (i.e., the viral protein).

The term "mutant" is used to mean a polypeptide having a primary amino acid sequence which differs from the wild-type sequence by one or more amino acid additions, substitutions or deletions. A mutant may arise naturally, or may be created artificially (for example by site-directed mutagenesis). Preferably the mutant has at least 90% sequence identity with the wild type sequence. Preferably the mutant has 20 mutations or less over the whole wild-type sequence. More preferably the mutant has 10 mutations or less, most preferably 5 mutations or less over the whole wild-type sequence.

The term "variant" is used to mean a naturally occurring polypeptide which differs from a wild-type sequence. A variant may be found within the same viral strain (i.e. if there is more than one isoform of the protein) or may be found within a different strains. Preferably the variant has at least 90% sequence identity with the wild type sequence. Preferably the variant has 20 mutations or less over the whole wild-type sequence. More preferably the variant has 10 mutations or less, most preferably 5 mutations or less over the whole wild-type sequence.

Here, the term "homologue" means an entity having a certain homology with the wild type amino acid sequence and the wild type nucleotide sequence. Here, the term "homology" can be equated with "identity".

In the present context, an homologous sequence is taken to include an amino acid sequence which may be at least 75, 85 or 90% identical, preferably at least 95 or 98% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, an homologous sequence is taken to include a nucleotide sequence which may be at least 75, 85 or 90% identical, preferably at least 95 or 98% identical to the subject sequence. Typically, the homologues will comprise the same sequences that code for the active sites etc. as the subject sequence. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e, one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al., 1984, Nucleic Acids Research 12:387). Examples of other software than can perform sequence comparisons include, but are not limited to, the BLAST package (see Ausubel et al., 1999 ibid - Chapter 18), FASTA (Atschul et al., 1990, J. Mol. Biol., 403-410) and the GENEWORKS suite of comparison tools. Both BLAST and FASTA are available for offline and online searching (see Ausubel et al., 1999 ibid, pages 7-58 to 7-60). However, for some applications, it is preferred to use the GCG Bestfit program. A new tool, called BLAST 2 Sequences is also available for comparing protein and nucleotide sequence (see FEMS Microbiol Lett 1999 174(2): 247-50; FEMS Microbiol Lett 1999 177(1): 187-8 and tatiana@ncbi.nlm.nih.gov).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). For some applications, it is preferred to use the public default values for the GCG package, or in the case of other software, the default matrix, such as BLOSUM62.

Once the software has produced an optimal alignment, it is possible to calculate % homology, preferably % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

The present invention also encompasses homologous substitution (substitution and replacement are both used herein to mean the interchange of an existing amino acid residue, with an alternative residue) may occur i.e. like-for-like substitution such as basic for basic, acidic for acidic, polar for polar etc. Non-homologous substitution may also occur i.e. from one class of residue to another or alternatively involving then inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids including; alpha* and alpha-disubstituted* amino acids, N-alkyl amino acids*, lactic acid*, halide derivatives of natural amino acids such as trifluorotyrosine*, p-Cl-phonylalanine*, p-Br-phenylalanine*, p-I-phenylalanine*, L-allyl-glycine*, β-alanine*, L-α-amino butyric acid*, L-γ-amino butyric acid*, L-α-amino isobutyric acid*, L-ε-amino caproic acid^{#}, 7-amino heptanoic acid*, L-methionine sulfone^{#*}, L-norleucine*, L-norvaline*, p-nitro-L-phenylalanine*, L-hydroxyprotine^{#}, L-thioproline*, methyl derivatives of phenylalanine (Phe) such as 4-methyl-Phe*, pentamethyl-Phe*, L-Phe (4-amino)^{#}, L-Tyr (methyl)*, L-Phe (4-isopropyl)*, L-Tic (1,2,3,4-tetrahydroisoquinoline-3-carboxyl acid)*, L-diaminopropionic acid^{#} and L-Phe (4-benzyl)*. The notation * has been utilised for the purpose of the discussion above (relating to homologous or non-homologous substitution), to indicate the hydrophobic nature of the derivative whereas # has been utilised to indicate the hydrophilic nature of the derivative, #* indicates amphipathic characteristics.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

The term "fragment" indicates that the polypeptide comprises a fraction of the wild-type amino acid sequence. It may comprise one or more large contiguous sections of sequence or a plurality of small sections. The polypeptide may also comprise other elements of sequence, for example, it may be a fusion protein with another protein. Preferably the polypeptide comprises at least 50%, more preferably at least 65%, most preferably at léast 80% of the wild-type sequence.

The mutant, variant, homologue or fragment rabies G sequence is capable of conferring the capacity for retrograde transport on the vector system.

A potential advantage of using the rabies glycoprotein is the detailed knowledge of its toxicity to man and other animals due to the extensive use of rabies vaccines. In particular phase 1 clinical trials have been reported on the use of rabies glycoprotein expressed from a canarypox recombinant virus as a human vaccine (Fries et al., 1996 Vaccine 14, 428-434), these studies concluded that the vaccine was safe for use in humans.

### EOIs/NOls

The present invention relates to the use of a vector system that is capable of transporting an entity of interest ("EOI").

The EOI may be a chemical compound, a biological compound or combinations thereof. By way of example, the EOI may be a protein (such as a growth factor), a nucleotide sequence, an organic and/or an inorganic pharmaceutical (such as an analgesic, an anti-inflammatory, a hormone, a lipid), or combinations thereof.

Preferably the EOI is one or more NOIs (nucleotide sequences of interest).

The vector system used in the present invention is a lentiviral vector system. It is possible to manipulate the viral genome so that viral genes are replaced or supplemented with one or more NOIs which may be heterologous NOIs.

The term "heterologous" refers to a nucleic acid or protein sequence linked to a nucleic acid or protein sequence to which it is not naturally linked.

In the present invention, the term NOI includes any suitable nucleotide sequence, which need not necessarily be a complete naturally occurring DNA or RNA sequence. Thus, the NOI can be, for example, a synthetic RNA/DNA sequence, a recombinant RNA/DNA sequence (i.e. prepared by use of recombinant DNA techniques), a cDNA sequence or a partial genomic DNA sequence, including combinations thereof. The sequence need not be a coding region. If it is a coding region, it need not be an entire coding region. In addition, the RNA/DNA sequence can be in a sense orientation or in an anti-sense orientation. Preferably, it is in a sense orientation. Preferably, the sequence is, comprises, or is transcribed from cDNA.

The retroviral vector genome may generally comprise LTRs at the 5' and 3' ends, suitable insertion sites for inserting one or more NOI(s), and/or a packaging signal to enable the genome to be packaged into a vector particle in a producer cell. There may even be suitable primer binding sites and integration sites to allow reverse transcription of the vector RNA to DNA, and integration of the proviral DNA into the target cell genome. In a preferred embodiment, the retroviral vector particle has a reverse transcription system (compatible reverse transcription and primer binding sites) and an integration system (compatible integrase and integration sites).

The NOIs may be operatively linked to one or more promoter/enhancer elements. Transcription of one or more NOI may be under the control of viral LTRs or alternatively promoter-enhancer elements can be engineered in with the transgene. Preferably the promoter is a strong promoter such as CMV. The promoter may be a regulated promoter. The promoter may be tissue-specific or cell-specific. In a preferred embodiment the promoter is neuron-specific. Especially preferred are promoters which restrict expression to C and/or Aδ fibres. In this way it is possible to avoid gene expression in the larger myelinated fibres which are responsible for transmission of other sensory stimuli.

Expression of the NOI may be inducible. Transcription of the NOI may thus be controlled, for example to modulate effective analgesia in pain applications. Inducible promoters include those regulated by hormones and hormone analogs such as progesterone, ecdysone and glucocorticoids as well as promoters which are regulated by tetracycline, heat shock, heavy metal ions, and lactose operon activating compounds.

The EOI/NOI may be or encode a protein of interest ("POI"). In this way, the vector delivery system could be used to examine the effect of expression of a foreign gene on the target cell. For example, the retroviral delivery system could be used to screen a cDNA library for a particular effect on the sensory neuron (or an alternative target cell).

The EOI/NOI may be or encode a cytoplasmic protein, nuclear protein, membrane protein, or secreted protein.

The EOI/NOI may be capable of integrating in the genome of a target cell.

The EOI/NOI may be capable of blocking or inhibiting the expression of a gene in the target cell. For example, the NOI may be an antisense sequence or an siRNA. The inhibition of gene expression using antisense technology is well known.

In one embodiment, the NOI comprises an siRNA. Post-transcriptional gene silencing (PTGS) mediated by double-stranded RNA (dsRNA) is a conserved cellular defence mechanism for controlling the expression of foreign genes. It is thought that the random integration of elements such as transposons or viruses causes the expression of dsRNA which activates sequence-specific degradation of homologous single-stranded mRNA or viral genomic RNA. The silencing effect is known as RNA interference (RNAi). The mechanism of RNAI involves the processing of long dsRNAs into duplexes of 21-25 nucleotide (nt) RNAs. These products are called small interfering or silencing RNAs (siRNAs) which are the sequence-specific mediators of mRNA degradation. In differentiated mammalian cells dsRNA >30bp has been found to activate the interferon response leading to shut-down of protein synthesis and non-specific mRNA degradation (Stark et al 1998). However this response can be bypassed by using 21 nt siRNA duplexes (Elbashir et al 2001, Hutvagner et al 2001) allowing gene function to be analysed in cultured mammalian cells.

The EOI/NOI or a sequence derived from the NOI may be capable of "knocking out" the expression of a particular gene in the target cell. There are several "knock out" strategies known in the art. For example, the NOI may be capable of integrating in the genome of the target cell so as to disrupt expression of the particular gene. The NOI may disrupt expression by, for example, introducing a premature stop codon, by rendering the downstream coding sequence out of frame, or by affecting the capacity of the encoded protein to fold (thereby affecting its function).

Alternatively, the EOI/NOI may be capable of enhancing or inducing ectopic expression of a gene in the target cell. The NOI or a sequence derived therefrom may be capable of "knocking in" the expression of a particular gene.

The EOI may be or encode a protein which has a therapeutic effect. For example, an NOI delivered by the vector delivery system may be a therapeutic gene - in the sense that the gene itself may be capable of eliciting a therapeutic effect or it may code for a product that is capable of eliciting a therapeutic effect.

In accordance with the present invention, suitable EOIs include those that are (or can produce) entities of therapeutic and/or diagnostic application such as, but not limited to: cytokines, chemokines, hormones, antibodies, anti-oxidant molecules, engineered immunoglobulin-fike molecules, a single chain antibody, fusion proteins, enzymes, immune co-stimulatory molecules, immunomodulatory molecules, anti-sense RNA, siRNA, a transdominant negative mutant of a target protein, a toxin, painal toxin, an antigen, a tumour suppresser protein and growth factors, vasoactive proteins and peptides, anti-viral proteins, ribozymes, receptor proteins and ion channels and derivatives thereof. The EOI may be an NOI which encodes a member of this list.

The EOI/NOI may also be or encode an antiapoptotic factor or a neuroprotective molecule. The survival of cells during programmed cell death depends critically on their ability to access "trophic" molecular signals derived primarily from interactions with other cells. For example, the NOI may encode a neurotrophic factor, such as ciliary neurotrophic factor (CNTF) or glial cell-derived neurotrophic factor (GDNF) or it may be a gene involved in control of the cell death cascade (such as Bcl-2). This may be useful in therapeutic strategies involving arresting neuronal and glial cell death induced by injury, disease, and/or aging in humans.

Neurotrophic factors are proteins which promote the survival of specific neuronal populations. Many have other physiological effects on neurons as inducing morphological differentiation, enhancing nerve regeneration, and stimulating neurotransmitter expression. These properties suggest that neurotrophic factors are highly promising as potential therapeutic agents for neurological diseases. Glial cell line-derived neurotrophic factor (GDNF) will most likely be applied to peripheral sensory neurons, since GFPa3 (one of the GDNF receptors) receptor is expressed predominantly in nociceptive sensory neurons. It has been demonstrated recently that GDNF both prevents and reverses sensory abnormalities that developed in neuropathic pain models. This effect is thought to occur as a consequence of the reversal by GDNF of the injury-induced plasticity of several sodium channel subunits. In a preferred embodiment, the EOI may be capable of biasing the transcriptome and/or proteotome of a cell. For example, the EOI may be one which is known to modulate gene expression in a given cell (such as a neuron). CREB (cyclic AMP element binding protein) is one such protein (Kornhauser et al., (2002) Neuron 34-221-233). DREAM is thought to act as a suppressor of transcription, for example of endogenous opiates (Cheng et al., 2002. Cell 108:31-43). Alternatively the EOI may act further upstream in the pathway. For example, the EOI may modulate the activity of a second entity which has the capacity to modulate gene expression. ERK MAP kinase has the capacity to activate CREB, and as such is an example of an "upstream factor" (Ji et al., (2002). J. Neurosci. 22:478-85).

The present invention also relates to a method of screening EOIs for their potential in preventing and/or treating and/or relieving pain. The present invention provides a screening method for identification and/or validation of an EOI useful in the prevention and/or treatment of pain which comprises the following steps:
(i) administration of a lentiviral vector system as described herein to a non-human subject such that it delivers a test EOI to a target cell in the subject by the method according to the first aspect of the invention, wherein the target cell is in situ within the DRG;
(ii) analysis of effect of the EOI on the target cell; and
(iii) selection of an EOI with therapeutic potential.

A test EOI may be screened individually, or a plurality of test EOIs may be screened simultaneously or sequentially.

### CELLULAR EXCITABILITY AND ION CHANNELS

In a highly preferred embodiment the EOI is capable of modulating the cellular excitability of a target cell. For example, the EOI may be capable of causing hyperpolarisation of the target cell.

As mentioned above, ion channels in the membrane of cells can open, allowing specific ions to diffuse across the plasma membrane down their electro-chemical gradient, to modulate the membrane potential of an excitable cell. Examples of such ion channels include: potassium channels, sodium channels, calcium channels and chloride channels.

The permeability of the cell membrane to potassium ions is particularly important. The resting membrane potential of a cell is an approximation of the potassium equilibrium potential. It is fundamentally dependent upon the permeability of the cell membrane to potassium ions. Over-activity of potassium channels leads to cell hyperpolarization and potassium channel openers (minoxidil) are in current therapeutic use, for example to cause a decrease in vascular tone. Activation of sodium or calcium channels and in some cases chloride channels, on the other hand, results in a net inward current (movement of net positive charge) and cell depolarisation.

Preferably the EOI is capable of modulating the expression and/or activity of an ion channel. An ion channel is a protein which allows an ionic current to flow across a membrane. For example, the EOI may be capable of modulating the expression of a potassium channel, a sodium channel, a calcium channel, a chloride channel and/or a non-selective cation channel.

In a highly preferred embodiment, the EOI is capable of causing the expression of a potassium channel or part thereof. The potassium channel may be constitutively active.

Examples of species that may be used to modulate membrane potential include (but are not limited to):
1. Ion channel subunits (native)
2. Ion channel subunits (mutant - see below)
3. Peptide activators of ion channels, for example βγ subunits of the G protein family have been shown to activate potassium channels in the heart.
4. Peptide blockers of ion channels
5. Antisense sequences or siRNAs which are capable of inhibiting the expression of ion channels

Mutant ion channels or subunit thereof may be constitutively active. Alternatively, they may be modified to include a gate which is controlled by an endogenous, or more preferably an exogenous signal (such as a drug-induced activator). The mutant channel or subunit may be modified to remove the control by an endogenous signal, for example by deleting the site responsible for control by PKC, PKA or pH.

### VOLTAGE-GATED SODIUM CHANNELS

Voltage-gated sodium channels play a central role in the initiation of action potentials in all neurons, therefore they represent a major target in the prevention of this hyperexcitability. The increase of excitability or variation of the baseline sensitivity in the primary sensory neurons leads to abnormal signal generation along the nociceptive pathway. In addition, several pharmacological approaches have demonstrated that specific sodium channel blockers, such as anti-convulsants, antidepressants and local anaesthetics may be effective tor the treatment of pain.

Multiple distinct sodium channels encoded by different genes (Type I, II, III, SM1, SM2, Na6, PN1, SNS2) are present in DRG. Some of these sodium channels are sensory neuron-specific and expressed in specific subpopulations of nociceptors (PN1, SNS, NaG). Two types of sodium channel have been differentiated on the basis of their kinetic and sensitivity to the neurotoxin tetrodotoxin (TTX). The fast-inactivating TTX-sensitive currents, are found in all DRG cells. The slowly inactivating TTX-resistant currents appear to be preferentially expressed in specific subpopulation of sensory neurons and are sensory neuron specific. Sodium channel expression within DRG neurons changes both during development, and also in various pain states. A loss of TTX-resistant currents has been described after axonal transection and expression of SNS is down-regulated. TTX-resistant sodium currents are however upregulated during inflammation.

Sodium channels have marked voltage sensitivity, and are composed of a principal alpha subunit and one or more smaller beta subunits. Only a single alpha subunit is required for channel activity and the subunit is four times as big as a 6tm Kv channel (equivalent to all four subunits being in one gene). There are c.10 genes, SCN1A, SCN2A, SCN3A, SCN4A, SCN5A, SCN6A, SCN7A, SCN8A, SCN1B1, SCN1B2. The beta subunit only has one single tm domain and modifies the alpha subunit current.

In a preferred embodiment the EOI is capable of modulating the expression or activity of voltage-gated sodium channels.

### POTASSIUM CHANNELS

Potassium channels fall into two main structural families: those having 6 or 2 transmembrane domains. There are six conserved families with 6 tm regions including voltage gated, KCNQ, and eag-like channels as well as three families of Ca-activated channels. The channels of the three named families are generally closed at the resting potential but open at depolarized potentials and are involved in events such as repolarization.

### 6tm Families

Kv Shaker (Kv1), Shab (Kv2), Shaw (Kv3), Shal (Kv4) and, Shaker divided into Kv1.1 - Kv1.9.
KCNQ KCNQ1, 2, 3
Eag eag, erg, elk
BK Sio, nSlo2
SK SK1, 2, 3
IK

The voltage gated K channels are made up of pore forming alpha subunits which may be associated with one of a number of beta subunits. Four alpha subunits are required for a functional channel. The beta subunits are cytoplasmic and modify alpha subunit expression and channel activity.

In the Shaker alpha subunit, at the distal end of the N terminus, there are a number of residues that act as an inactivation ball by plugging the pore.

### KIR Channels

These channels stabilize the resting membrane potential near the K equilibrium potential and show strong inward rectification (pass less outward current in response to a hyperpolarizing potential step than inward current in response to a depolarizing step of the same amplitude. There are six main subfamilies, and there are a number of different isoforms and splice variants for each gene. As with the Kv channels, KIR can form heteromultimers which affects currents.
Kir 1.1(ROMK1), 4.1, 6.1, 6.2(KATP), 2.1 (IRK), 2.2, 2.3, 3.2(GIRK2),
   3.4(GIRK4), 3.3(GIRK3), 3.1 (GIRK1), 5.1 (old names given in parentheses)
KIR 6.1 and 6.2 do not form functional channels without the sulphonyl urea receptor (SUR1 and SUR2).

In a preferred embodiment the EOI is capable of modulating the expression or activity of a potassium channel.

### CALCIUM CHANNELS

The six types of calcium channel, T, L, N, P, Q, R and are distinguished by their sensitivity to pharmacological blockers. They are composed of many different subunits which coassemble. For example, in skeletal muscle the L-type channels are made up from alpha1, 2, beta, gamma and delta subunits in a 1.1.1.1.1 stoichiometry.

In a preferred embodiment the EOI is capable of modulating the expression or activity of a calcium channel.

### ASICs

Acid-sensing ion channels (ASICs) are members of the amiloride sensitive sodium channel/ENaC family of ion channels. They are expressed in the central nervous system and in sensory neurons and are activated by extracellular protons. Painful conditions such as ischaemia and inflammatory disorders are associated with tissue acidosis (pH7 - pH5) with the sensation being a result of the direct action of protons on ASICs.

Given the number of different insults that may contribute to the initiation of the painful stimulus other than those associated with tissue acidosis, it is more effective to inhibit the transmission of the action potential (e.g. at the level of the DRG) rather than to inhibit a potential component of the initial signal generation. In this sense, ASICs merely act as the receptors for the initiation of the action potential and are not specific targets. In contrast, preferred targets for the present invention are those that allow general inhibition of cellular excitability or of propagation of the action potential and as such will decrease the transmission of any number of painful stimuli. Accordingly, in preferred embodiments of the invention, the EOI is capable of modulating the expression or activity of a voltage -gated sodium channel, a potassium channel or a calcium channel and not an ASIC.

### RECEPTORS

Alternatively, the EOI may be capable of modulating the expression or activity of a receptor, in particular a receptor found on cells which are located wholly or partly within the DRG.

Opioids, receptors for which are located on peripheral and central neurons, are the treatment of choice during acute post-operative pain. In nerve injury, the down-regulation of opioid receptors, especially µ-receptors in DRG could explain the ineffectiveness of opioids treatment in neuropathic pain. More recent studies suggest that a specific population of the rostroventromedial medulla (RVM) neurons, those expressing opioid µ-receptors, is critical in the behavioural expression of experimental animals. Neurons in the RVM project to the spinal cord loci where the neurons inhibit or facilitate pain transmission. Inhibition of neuropathic pain has been shown by selective ablation of RVM cells expressing the µ-opioid receptor in spinal nerve ligation injury model. This result suggests that the descending projections from the brainstem have an important role in facilitating pain transmission.

In order to treat pain, the vector system of the present invention may be used to deliver an EOI which is capable of inhibiting expression of µ-receptors, particularly in RVM neurons in the brain. For example, the EOI may be or encode an antisense sequence for µ-receptors. Conversely the EOI may be capable of causing the overexpression of opioid receptors for the treatment of conditions in which opioid receptors are down-regulated. If the number of opioid receptors in the DRG was increased, this may enhance the effectiveness of pain relief by administration of opioids.

Pain hypersensitivity is largely an expression of changes in the excitability of neurons of the spinal cord with alteration of the properties of the NMDA receptors. NMDA antagonists, such as ketamine or dextrometorphan have been demonstrated to be effective in neuropathic pain treatment. If NMDA receptors are involved in pain, it should be possible to control such pain if the EOI is or encodes an entity which blocks the expression or activity of such receptors.

The EOI may be capable of modulating the activity or expression of an antinociceptive target, such as an NK1 receptor, PCK-γ, VR1 receptor, NMDA receptor or an N-type calcium channel. For example, the EOI may encode antisense or si RNA sequences against these receptors.

The EOI may be capable of modulating the activity or expression of a pronociceptive target, such as a CB_{1/2} receptor, PCK- y, mACH receptor, nACH receptor, opioid receptor or an α2 adrenergic receptor.

### TARGET CELL

The EOI is capable (directly or indirectly) of exerting an effect on a target cell. If the EOI is delivered to a sensory neuron within the DRG, this is the target cell. Following delivery to the sensory neuron, the EOI (in the same or a different form i.e. optionally modified) may move on to a different target cell.

For example, the EOI may be an NOI which is capable of encoding a secretable protein. Once secreted, the protein may exert an effect on a target cell (for example a neighbouring cell). For some applications, the NOI expression product may demonstrate a general bystander effect; that is the production of the expression product in one cell leading to the modulation of additional, related cells, either neighbouring or distant, which possess a common phenotype.

The target cell may be a sensory neuron. Alternatively the target cell may be a different cell type found in the DRG, such as a glial cell, Preferably the target cell neighbours the cell body of the sensory neuron which receives the EOI.

The target cell is a cell within the DRG. Apart from sensory neurons (the cell bodies for which are within the DRG), the DRG also comprises glial cells.

Preferably the target cell is a sensory neuron. Especially preferred is a sensory neuron either within a C fiber or an Aδ fiber. C fiber neurons are especially preferred.

Sensory neurons are pseudo-unipolar neurons having a single process which projects from the cell body. This process bifurcates to form terminals in the periphery (the peripheral branch) and in the grey matter of the spinal cord where they synapse with other neurons (the central branch).

If the EOI is delivered to the cell body of a sensory neuron in the DRG it can then travel (with or without modification) to the spinal cord via the central branch. Once in the spinal cord the EOI or derivative (which may be a modified EOI) thereof can then exert an effect on other cells, such a motor neurons, or intemeurons.

The present invention also provides, therefore, the use of a vector system as defined herein for delivering an EOI to the spinal cord, which comprises the following steps:
(i) delivery of an EOI to the cell body of a sensory neuron;
(ii) optional modification of the EOI; and
(iii) delivery of the optionally modified EOI from the cell body of the sensory neuron to the spinal cord via the central branch of the sensory neuron.

The EOI may thus be modified by any suitable means. The nature of the modification will of course depend on the nature of the EOI but includes any alteration of the EOI which occurs in the cell body, including translation of the NOI to a POI (which may thus be the modified EOI), and processing steps in the Golgi apparatus, such as post-translational modification, glycosylation reactions etc.

For example, the vector system may deliver an NOI to the cell body of a sensory neuron. The NOI may be translated into a POI within the cell body and the POI delivered to the spinal cord via the central branch.

In this embodiment, the (optionally modified) EOI may, for example, be capable of modulating the activity or expression of a neurotransmitter, a neurotrophin (such as GDNF, BDNF, NT3, CTNF and nerve growth factor), an antiapoptotic factor, an ion channel and or a receptor.

This method may be used for non-invasive access to the CNS, and so it is suitable for the treatment and/or prevention of any condition which affects the brain and/or spinal cord. These include conditions associated with motor neurons, such as motor neuron disease. For example, Amyotrophic Lateral Sclerosis (ALS) may be treatable with the use of anti-apoptotic factors. Spinal muscular atrophy (in neonates) may be preventable or treatable by replacing survival motor neuron gene 1, in order to avoid apoptosis. These also include other conditions associated with sensory neurons. For example encephalins may be used to regrow sensory neurons in conditions such as paraplegia.

### DRG

Spinal cord organisation appears to be segmented because the 31 pairs of spinal nerves emerge at regular intervals. Spinal nerves are the paths of communication between the spinal cord and the nerves innervating specific regions of the body. Two bundles of axons, called "roots", connect each spinal nerve to a segment of the cord.

The dorsal root contains sensory fibres, which conduct impulses from the periphery to the central nervous system. Each dorsal root has a swelling, the dorsal root ganglion (DRG) which contains the cell bodies of sensory neurons. The ventral root contains axons of motor neurons, which conduct impulses from the CNS to effector organs and cells.

Sensory neurons originate in the DRG. Different DRGs provide a somatosensory representation of the body. The area of skin innervated by a single dorsal root is called a dermatome. Topographical maps are available of the DRGs within the human body.

By administration of the vector system such that it delivers an EOI to a DRG of the subject, the EOI is delivered to a particular subset of sensory neurons.

The present invention also provides a screening method for identifying and/or validating an EOI with potential for pain relief. In the method, the effect of a test EOI on a target cell is monitored. The target cell is a cell of the DRG in situ.

### PAIN

The present invention relates to a vector system for use in the manufacture of a medicament for use in a method for treating and/or preventing pain.

Pain can be classified into two major types, fast and slow. Fast pain has been described as sharp, acute, pricking or electric while slow pain has been referred to as chronic, burning, aching, throbbing or nauseous. The nerve fibres that are responsible for pain transmission are the A∗ and C fibres. Fast pain is carried by A* fibres with conduction velocities of 6-30 m.s⁻¹. These fibres are activated by mechanical or thermal stimuli. By contrast, C fibres are activated either by chemical (slow-chronic) pain or by persistent mechanical/thermal stimuli and have conduction velocities of 0.5-2 m.s⁻¹.

Transmission of pain from the periphery into the central nervous system occurs through dual pathways - in the neospinothalamic and paleospinothalamic tracts. The neospinothalamic tract is predominantly formed by the A∗ fibres which terminate mainly in the lamina marginalis of the dorsal horn. Pain transmission in the paleothalamic tract is carried mainly, but not exclusively, by C fibres. These fibres terminate in the substantia gelatinosa. Both tracts pass through the anterior commissure and then pass upward to the brain in the anterolateral pathway before terminating in the reticular nuclei of the brainstem or thalamus.

The use of the vector system of the present invention is particularly suitable for the treatment of chronic intransient pain. Examples of such pain is that associated with conditions such as cancer, osteo and rheumatoid arthritis, back pain, sciatica and multiple sclerosis. The system is also useful for treating post-operative pain.

In a particularly preferred embodiment the use of the vector system of the present invention is for the treatment of neuropathic pain, a maladaptive form of pain which occurs after peripheral or central nervous system injury. Neuropathic pain is initiated or caused by a primary lesion or dysfunction of the nervous system. It includes diabetic neuropathy, cancer-related and HIV-related pain.

### ADMINISTRATION ROUTES

In the use of the present invention, the vector system travels from the administration site to the DRG by retrograde transport.

Administration to a site which is distant to the DRG is advantageous because access to the distal site may be easier than access to the DRG. Also, by using retrograde transport is it possible to deliver the EOI to certain cells or groups of cells. For example, where the vector system is administered peripherally at the site of pain, the vector system (or part thereof) will travel to the DRG by retrograde transport and deliver the EOI to cells which are directly involved in sensing the pain.

There are other administration sites which may be used for this embodiment of the invention which are easier to access than the DRG, such as the dorsal horn of the spinal cord, or the sciatic nerve. Injection into the DRG or sciatic nerve may be used to increase transduction in the DRG relative to a more distant site (injection into a footpad or the site of pain).

The vector system is pseudotyped with at least part of a rabies G protein or a mutant, variant, homologue or fragment thereof (see above), which renders it capable of travelling by retrograde transport.

### RETROGRADE TRANSPORT

The cell body is where a neuron synthesises new cell products. Two types of transport systems carry materials from the cell body to the axon terminals and back. The slower system, which moves materials 1-5mm per day is called slow axonal transport. It conveys axoplasm in one direction only (from the cell body toward the axon terminals (anterograde transport)). There is also "Fast transport" which is responsable for the movement of membranous organelles at 50-200 mm per day away from the cell body (anterograde) or back to the cell body (retrograde) (Hirokawa (1997) Curr Opin Neurobiol 7(5):605-614).

Vector systems comprising rabies G protein are capable of retrograde transport (i.e. travelling towards the cell body). The precise mechanism of retrograde transport is unknown, however. It is thought to involve transport of the whole viral particle, possibly in association with an intemalised receptor. The fact that vector systems comprising rabies G can be specifically transported in this manner (as demonstrated herein) suggests that the env protein may be involved.

HSV, adenovirus and hybrid HSV/adeno-associated virus vectors have all been shown to be transported in a retrograde manner in the brain (Horellou and Mallet (1997) Mol Neurobiol 15(2) 241-256; Ridoux et al (1994) Brain Res 648:175-175; Constantini et al (1999) Human Gene Therapy 10:2481-2494). Injection of Adenoviral vector system expressing glial cell line derived neurotrophic factor (GDNF) into rat striatum allows expression in both dopaminergic axon terminals and cell bodies via retrograde transport (Horellou and Mallet (1997) as above; Bilang-Bleuel et al (1997) Proc. Natl. Acd. Sci. USA 94:8818-8823).

Retrograde transport can be detected by a number of mechanisms known in the art. For example, it is known to monitor labelled proteins or viruses and directly monitor their retrograde movement using real time confocal microscopy (Hirokawa (1997) as above).

In the present invention, the vector system (or part thereof) is capable of travelling from the administration site to the DRG by retrograde transport. In a preferred embodiment, the vector system travels up the axon of a sensory neuron, to the cell body (within the DRG).

In a preferred embodiment the vector system is administered to a peripheral administration site. The vector may be administered to any part of the body from which it can travel to the DRG by retrograde transport. In other words the vector may be administered to any part of the body to which a sensory neuron projects.

The "periphery" can be considered to be all part of the body other than the CNS (brain and spinal cord). In particular, peripheral sites are those which are distant to the CNS. Sensory neurons may be accessed by administration to any tissue which is innervated by the neuron. In particular this includes the skin, muscles and the sciatic nerve.

An advantage with the peripheral administration system is that it is possible to target particular groups of cells (e.g. sets of neurons), or a particular neural tract by choosing a particular administration site. Where a subject is suffering from pain (in particular slow, chronic pain), the particular sensory neuron(s) involved in transmitting the pain may be targeted by administration of the vector system directly into the area of pain.

### SCREENING METHODS

The present invention provides a screening method for identifying and/or validating new drugs for use in pain therapy.

For example, there is provided a screening method for identifying new EOI(s) which are useful in the prevention and/or treatment of pain.

There is provided a screening method for identification and/or validation of an EOI useful in the prevention and/or treatment of pain which comprises the steps of
(i) administration of a lentiviral vector system as defined herein to a non-human subject such that it delivers a test EOI to a target cell in the subject by the method according to the first aspect of the invention, wherein the target cell is in *situ* within the DRG;
(ii) analysis of effect of the EOI on the target cell; and
(iii) selection of an EOI with therapeutic potential.

The term "test EOI" is used to indicate a candidate EOI whose usefulness in the prevention and/or treatment of pain is under investigation. The test EOI may be a single entity or it may be one of a plurality of compounds being tested either simultaneously or sequentially.

In a preferred embodiment, the EOI affects transcription/translation of one or more genes in the target cell. For example, the EOI may be an antisense sequence which binds the nascent transcript, which may cause its degradation (e.g. by RNAse H) and/or block its translation. In another embodiment, the EOI may be an siRNA. Transcription and/or translation of a given gene may be detected by a number of methods known in the art. The presence or absence of a particular RNA may be detected, for example, by RT-PCT, Northern blotting or In situ hybridisation. The protein encoded by the gene may be detected by Western blotting or, if an antibody specific for the protein is available, ELISA or FACS analysis.

The EOI may affect expression of one or more genes. The pool of RNAs expressed in a cell is sometimes referred to as the transcriptome. Methods for measuring the transcriptome, or some part of it, are known in the art. A collection of article summarizing some current methods appeared as a supplement to the journal Nature Genetics. (The Chipping Forecast. Nature Genetics supplement, volume 21, January 1999.) A preferred method for measuring expression levels of mRNAs is to spot PCR products corresponding to a large number of specific genes on a nylon membrane such as Hybond N Plus (Amersham-Pharmacia). Total cellular mRNA is then isolated, labeled by random oligonucleotide priming in the presence of a detectable label (e.g. alpha 33P labeled radionucleotides or dye labeled nucleotides), and hybridized with the filter containing the PCR products. The resulting signals can be analyzed by commercially available software, such as can be obtained from Clontech/Molecular Dynamics or Research Genetics, Inc.

Experiments have been described in model systems that demonstrate the utility of measuring changes in the transcriptome before and after changing the growth conditions of cells, for example by changing the nutrient environment. The changes in gene expression help reveal the network of genes that mediate physiological responses to the altered growth condition. Similarly, the addition of a drug to the cellular or in vivo environment, followed by monitoring the changes in gene expression can aid in identification of gene networks that mediate physiological and pharmacological responses. A similar approach could be used to screen EOIs which, for example, act as growth factors.

EOIs which bias the transcriptome in neurons are known in the art. For example, increased intracellular calcium concentrations or ERK MAP kinase are thought to activate CREB (cyclic AMP element binding protein) to modulate gene expression. (Kornhauser et al., (2002) Neuron 34:221-233; Ji et al., (2002) J. Neurosci. 22:478-85). Also, DREAM is thought to act as a suppressor of transcription of endogenous opiates. (Cheng et al., (2002). Cell 108:31-43). The EOI of the present invention may have a similar capacity to bias the transcriptome or it may a gene whose expression is controlled (i.e. repressed or activated) by such an EOI. Either may be detected using a screening method of the present invention.

Thus, novel genes may be identified as a result of their capacity to modulate the transcriptome/proteotome or as a result of EOI-induced modulation of their transcription/translation. For example, a gene could be identified by expressing ERK MAP kinase , CREB or DREAM (possibly in a constitutively active form) and examining the resulting change in gene expression.

The pool of proteins expressed in a cell is sometimes referred to as the proteome. Studies of the proteome may include not only protein abundance but also protein subcellular localization and protein-protein interaction. Methods for measuring the proteome, or some part of it, are known in the art. One widely used method is to extract total cellular protein and separate it in two dimensions, for example first by size and then by isoelectric point. The resulting protein spots can be stained and quantitated, and individual spots can be excised and analyzed by mass spectrometry to provide definitive identification. The results can be compared from two target cell samples, only one of which has been treated with the EOI.

The differential up or down modulation of specific proteins in response to EOI treatment may indicate their role in mediating the physiological and pharmacological actions of the EOI. Another way to identify the network of proteins that mediate the actions of the EOI is to exploit methods for identifying interacting proteins. This approach, which is known as proteomics, is well known in the art (see, for example, Blackstock et al. Proteomics: quantitative and physical mapping of cellular proteins. Trends Biotechnol. 17 (3): p. 121-7, 1999; Patton W. F., Proteome analysis II. Protein subcellular redistribution: linking physiology to genomics via the proteome and separation technologies involved. J Chromatogr B Biomed Sci App. 722(1-2):203-23. 1999.)

The EOI may exert an effect on the excitability of the target cell. For example, the EOI may block the action of one or more ion channels in the target cell. Methods for modulating the excitability of cells *in vitro* are well known in the art (Lanigan et al (2001) Biochemistry 40:15528-37; Braun et al (2000) J Physiol. 527:479-92).

The present invention also relates to methods for identifying and/or validating a therapeutic EOI in vivo. Such methods involve the administration of a vector system capable of delivering the test EOI to a subject such that it is delivered to the DRG. The subject is then analysed to see if the EOI has the desired effect in preventing and/or alleviating pain.

Analysis of pain avoidance or relief may be accomplished by a number of known methods. Changes in transmission can be measured by standard electrophysiological techniques in vitro. See for example Hamill et al (1981) Pflugers Arch 391:85-100. Methods for evaluating pain in vivo include the hot plate, tail flick, and formalin tests. See Current Protocols in Neuroscience. Volume 2, Chapter 8.9 Published by John Wiley and Sons.

Preferably the method of the present invention involves an *in vitro* identification step, followed by an *in vivo* verification step. Such a method may, for example, involve:
(i) selection of an EOI with therapeutic potential by analysing its effect on an *in vitro* target cell;
(ii) delivery of the selected EOI to the DRG of a subject; and
(v) analysis of pain in the subject.

This two-step approach facilitated the in vitro screening of a number of candidate drugs, followed by *in vivo* testing of those which show potential. If the drug then shows promise in the *in vivo* (for example, animal) screen, then it can go on to clinical trials to assess its usefulness for treating humans and safety.

### PHARMACEUTICAL COMPOSITIONS

The present invention also provides the use of a vector delivery system in the manufacture of a pharmaceutical composition. The pharmaceutical composition may be used to deliver an EOI, such as an NOI, to the DRG of a subject

The vector delivery system is a lentiviral vector delivery system.

The pharmaceutical composition may be used for treating an individual by gene therapy, wherein the composition comprises or is capable of producing a therapeutically effective amount of a vector system according to the present invention.

The pharmaceutical composition may be used to treat a human or animal subject. Preferably the subject is a mammalian subject. More preferably the subject is a human. Typically, a physician will determine the actual dosage which will be most suitable for an individual subject and it will vary with the age, weight and response of the particular subject.

The composition may optionally comprise a pharmaceutically acceptable carrier, diluent, excipient or adjuvant. The choice of pharmaceutical carrier, excipient or diluent can be selected with regard to the intended route of administration and standard pharmaceutical practice. The pharmaceutical compositions may comprise as (or in addition to) the carrier, excipient or diluent, any suitable binder(s), lubricant(s), suspending agent(s), coating agent(s), solubilising agent(s), and other carrier agents that may aid or increase the viral entry into the target site (such as for example a lipid delivery system).

In the use of the vector system of the invention (where the vector system is administered to a site which is distant to the DRG and travels to the DRG by retrograde transport) it is convenient if the vector system is injected at the distant site.

The invention will now be further described by way of Examples, which are meant to serve to assist one of ordinary skill in the art in carrying out the invention and are not intended in any way to limit the scope of the invention. The Examples refer to the Figures:
Figure 1 shows dissociated DRG transduced with pONY8G,5'cPPT at MOI = 10 at DIV4. (a) shows expression of GFP and (b) shows the corresponding bright field image.
Figure 2 shows viral transfer of genes to sensory neurons. Expression of the reporter gene β-galactosidase in the dorsal root (A-C) and DRG (D, E) after injection of pONY8Z pseudotyped with rabies-G into the dorsal horn of the spinal cord. Sections showing immunofluorescence for β-galactosidase 5 weeks after viral injections. Expression of β-gal is detectable in Schwann cells, axons (block arrow) and DRG neurons (arrow).
Figure 3 shows expression of the reporter gene β-galactosidase in DRG neurons 4 weeks after pONY8Z vectors pseudotyped with rabies G envelope were injected into the footpad of 4 rats.
Figure 4 shows transduction of DRG neurons after intranerval injection of pONY8Z rabies-G. Sections showing immunofluorescence for β-galactosidase 5 weeks after viral injections (A). Same sections were stained for the neuronal marker NeuN (B). (C) shows composite image

### EXAMPLES

### Example 1- Expression of marker genes in dissociated and tissue explants of dorsal root ganglia.

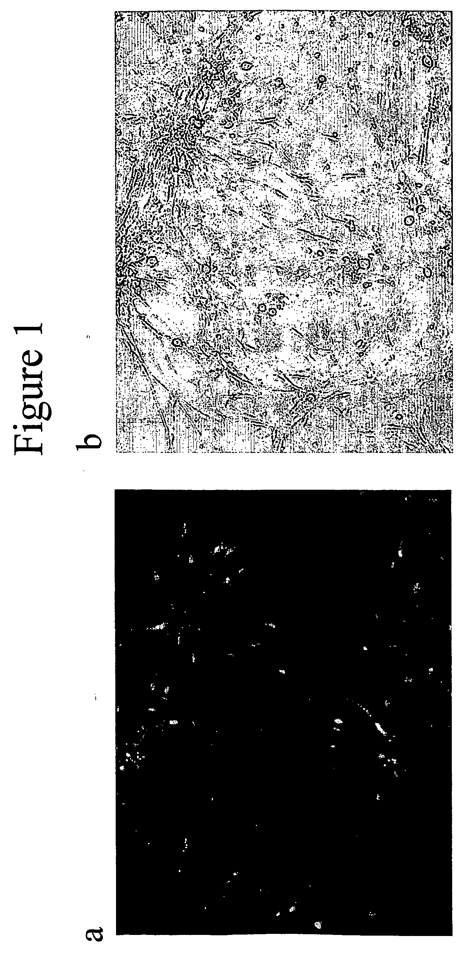

DRGs are isolated from E18 rats according to standard dissection protocols. Cells are dissociated in trypsin for 5 minutes before pelleting down and resuspending in plating media (DMEM + 10%FCS, & Gentamycin). Cells are plated out on poly-D-lysine treated glass chamber slides at 1000 cells/mm2 and maintained in a tissue culture incubator at 37 C for four days. On Day 1 *in vitro,* cells are transduced with pONY8G,5'cPPT (a virus capable of expressing green fluorescent protein from a CMV promoter) at an MOI of 10 in neurobasal medium (+B27) for five hours. Polybrene is added to the transduction medium at a concentration of 2 ug/ml-1. After transduction, the media is replaced with fresh neurobasal media and the cells are kept for a further three days. On Day 4 in vitro, transduced cells (GFP positive) are visualized with a microscope equipped with a fluorescent light source and FITC filter set. At least 45% of the cells are transduced and are shown in Figure 1 a and b.

### Example 2 - Expression of marker genes in dorsal root glanglia after direct injection of the virus.

Dorsal root ganglia are surgically exposed by dissecting the musculus multifidus and the musculus longissimus lumborum and by removing the processus accessorius and parts of the processus transversus. An EIAV vector coding for the reporter gene β-gal is injected directly in the DRG. Subjects will receive 1 µl of the viral solution per ganglion. All injections are done by using a stereotaxic frame and a Hamilton syringe with 34-gauge needle. The solution is slowly infused at the speed of approximately 0.1 µl/min. To determine the transduction efficiency of EIAV vector for sensory neurons, histology and immunohistology using β-gal antibodies (Affiniti) is performed at different time points.

### Example 3 - Expression of marker genes in dorsal root ganglia after peripheral administration of the virus

The procedure of the application of the virus on the skin surface has been described previously (Wilson et al., 1999 Proc Natl Acad Sci USA, 96(6):3211-3216.). Briefly, the hair is removed from the dorsal of the hindfoot surface. The skin is scarified using medium-coarse sandpaper. Ten microliters of the viral solution is applied to each foot. The side of a pipettor tip is used to spread the virus. Alternatively, or additionally, the virus can be injected into the footpad. The virus is retrogradely transported to the DRG and can be detected using β-gal antibodies (5'-3').

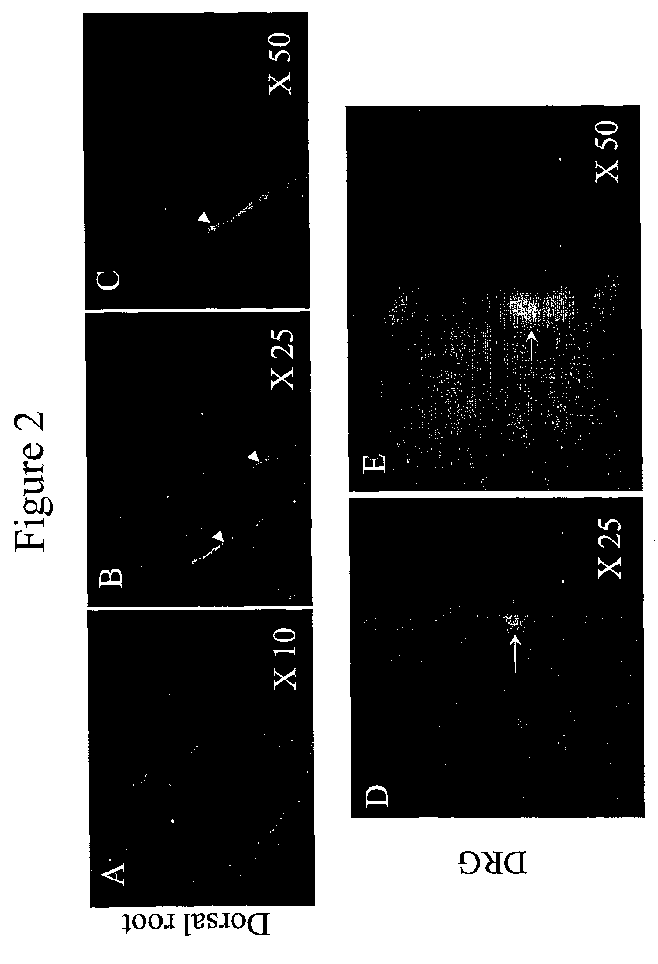

pONY8Z vectors were injected into the footpads of 8 rats and analysed 4 weeks post-transduction (rabies-G 6x10⁶ TU/ml (20µl), n = 4; VSV-G 6 x 10⁶ TU/ml (20µl), n = 4). Rabies-G pseudotyping confers retrograde transport of the viral vector (see Mazarakis et al., 2001. Human Molecular Genetics, 10:2109). Histological sections from the DRG at x40 magnification were examined. All animals displayed retrogradely transduced DRG neurons (Fig. 2 A-E). However, in contrast to pONY8Z rabies-G injected rats, no β-gal reactivity was detectable in DRG sections from rats injected with pONY8Z VSV-G.

### Example 4 - Expression of marker genes in dorsal root ganglia after direct injection into the spinal cord

Group of rats are injected with pONY8Z (rabies-G or VSV-G) or equivalent amount of PBS, via a posterior laminectomy within the dorsal horn of the spinal cord. Three injection sites at the lumbar level, separated by 2 mm, are performed. Each rat received 1 µl per site of the viral solution at dorso-ventral coordinate of 0.5 mm.

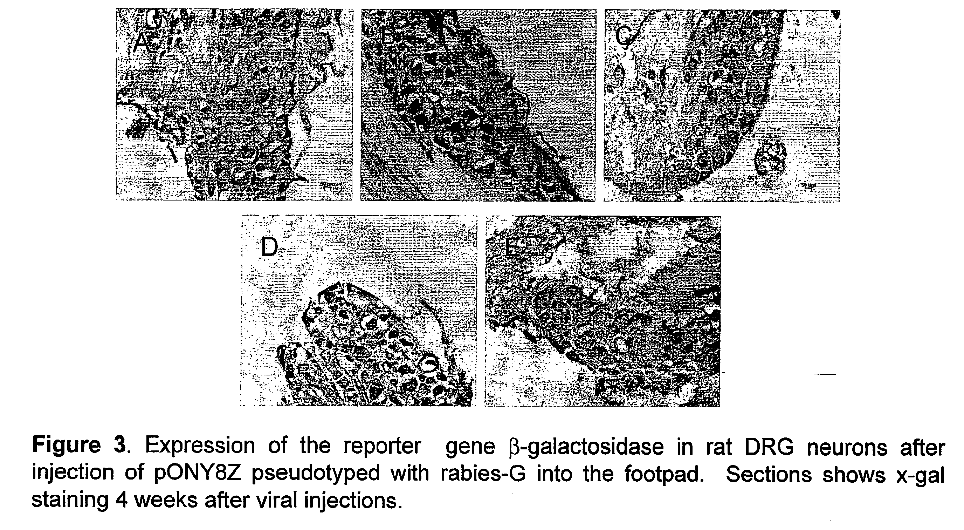

pONY8Z vectors were injected into the dorsal horn in four rats and analysed 5 weeks post-transduction (rabies-G 3.8 x 10⁸ TU/ml, n = 2; VSV-G 1.2 x 10⁹ TU/ml, n = 3). Rabies-G pseudotyping confers retrograde transport of the viral vector (see Mazarakis et al., 2001. Human Molecular Genetics, 10:2109). Histological sections from the spinal cord, the dorsal root and DRG were examined at various magnifications. All animals showed expression of the marker gene to the immediate neighbourhood of the site of injection into the spinal cord. Of 3 rats injected into the spinal cord with pONY8Z rabies-G, 2 showed expression of β-gal in Schwann cells. 4xonal expression also was seen (Fig. 3A-C). The two rats displayed retrogradely transduced DRG neurons (Fig. 3D-E). However, in contrast to pONY8Z rabies-G injected rats, no β-gal reactivity was detectable in dorsal root and DRG sections from rats injected with pONY8Z VSV-G.

### Example 5 - Expression of potassium channels in dissociated dorsal root ganglion cells.

Dissociated DRG cells are prepared as described in example 1. On Day 1 *in vitro,* cells are transduced with (Smart2Kir_IRES_GFP), av virus encoding the inward rectifier potassium channel (KIR6.2) and GFP. On DIV4 cells are visualized under fluorescent light and are used for patch clamp electrophysiology according to standard patch clamp electrophysiology procedures. Results show that cells transduced with virus have lower resting membrane potentials (hyperpolarisation).

### Example 6 - Expression of potassium channels in dorsal root ganglia after direct injection.

(Smart2Kir_IRES_GFP) is directly injected into rat DRG following the procedure of Example 2. Transduction is assessed using either by GFP fluorescence or antibody staining.

### Example 7 - Expression of potassium channels in dorsal root ganglia after peripheral delivery

(Smart2Kir_IRES_GFP) is injected into rats by subcutaneous injection in the hindfoot as described in example 3. Transduction is assessed using either GFP fluorescence or antibody staining.

### Example 8 - Expression of antisense message specific for sodium channels in DRG neurons after injection into the footpad.

Twenty microliters of the viral vectors (Smart2antisense-flag) is applied to each footpad. The virus is retrogradely transported to the DRG and can be detected using antibodies against Flag (Sigma).

### Example 9 - Expression of reporter gene in DRG neurons after intrasciatic injection

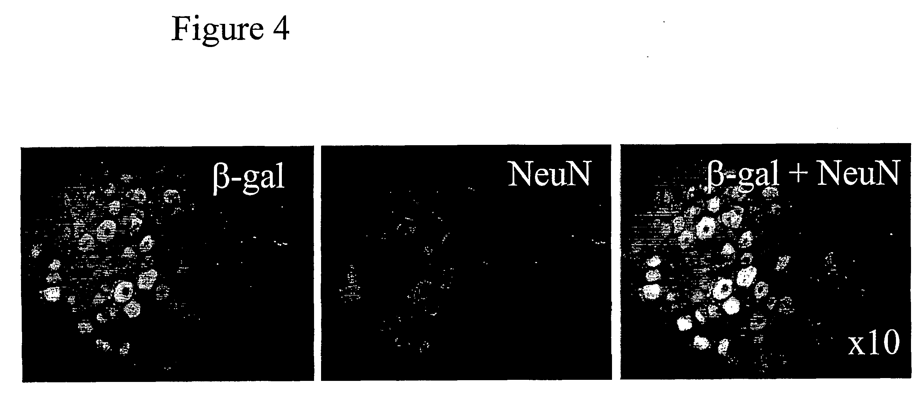

Adult rats (n = 3) were anaesthetized and injected with an pONY8Z Rabies-G pseudotyped viral vector (2 µl) into the sciatic nerve. Five weeks following sciatic injection of the viral vector, animals were sacrificed and dorsal root ganglia removed. Tissue was sectioned and immunolabelled for NeuN and for β-galactosidase. In order to visualize staining, Cy3 anti-mouse and Alexa-488 anti-rabbit secondary antibodies against the NeuN and β-galactosidase primary antibodies were used (Figures 4A and B). Double labelling of neuronal bodies in the dorsal root ganglia is demonstrated by colocalization of the two markers in yellow (Figure 4C).

### Example 10 - Expression of antisense message specific for sodium channels in DRG neurons after intrasciatic injection.

For intranerval injection, the right sciatic nerve of anaesthetized rat is surgically exposed. The nerve was gently placed onto a metal plate and Smart2Z or Smart2antisensesodimchannel-flag pseudotyped with rabies-G envelope are injected with a 33-gauge Hamilton syringe over 3 min. The volume injected per rat is 2-3 µl. The sciatic nerve is anatomically repositioned, and the skin was closed with vicryl 5/0 sutures.

### Example 11 - Expression of neurotrophic factor in the DRG neurons after intrasciatic injection.

Intranerval injection of vectors expressing neurotrophic factors is performed as described in example 10.

### Example 12 - Screening of a plurality of test compounds in vitro and in vivo

The gene encoding the calcium sensing protein DREAM is expressed in an EIAV viral vector in cultured dissociated dorsal root ganglia. Cells are dissected, cultured and transduced as described in previous examples. After transduction, RNA is isolated and compared with cells transduced with an empty viral vector. RNA are analysed using the Affymetrix chip system. Similarly, cells are transduced with a viral vector expressing a constitutively active version of the immediate upstream activator of both ERK1 and ERK2, mitogen-activated/extracellular signal-regulated kinase 1 (MEK1), to activate ERK signalling. Again by comparison of RNA using the Affymetrix system, changes in gene expression as a result of physiological processes that are associated with modulation of pain are analysed.

## Claims

1. The use of a lentiviral vector system pseudotyped with at least part of a rabies G protein or a mutant, variant, homologue or fragment thereof in the manufacture of a medicament for use in a method for treating and/or preventing pain in a subject, which method comprises the step of administration of the lentiviral vector system such that it delivers an Entity of Interest (EOI) to a Dorsal Root Ganglion (DRG) of the subject, wherein
(i) the vector system or part thereof travels from the administration site to the DRG by retrograde transport; and
(ii) said pseudotyping entity has the activity of enabling the vector system to travel by retrograde transport to the DRG.

2. The use according to claim 1, wherein the administration site is a peripheral site.

3. The use according to claim 1 or 2, wherein the vector system is administered to the subject by injection into the area of pain.

4. The use according to any preceding claim, wherein the EOI is capable of modulating the cellular excitability of a target cell.

5. The use according to claim 4, wherein the EOI is capable of causing hyperpolarisation of the target cell.

6. The use according to any preceding claim, wherein EOI is capable of modulating the expression and/or activity of an ion channel.

7. The use according to claim 6, wherein EOI is capable of causing the expression of an ion channel or part thereof.

8. The use according to claim 7, wherein the ion channel is constitutively active.

9. The use according to any preceding claim, in which vector system:
(iii) the EOI is a nucleotide sequence of interest (NOI);
(iv) expression of the NOI is under the control of a targeted promoter; and
(v) the targeted promoter restricts the expression of the NOI to C fibers and/or Aδ fibres.

10. The use according to any of claims 1 to 8, in which vector system:
(i) the EOI is an NOI; and
(ii) expression of the NOI is inducible.

11. The use according to any preceding claim, wherein the EOI is delivered to the cell body of a sensory neuron within the DRG.

12. The use according to claim 11, for delivering an EOI to the spinal cord, which comprises the following steps:
(i) delivery of an EOI to the cell body of a sensory neuron within the DRG;
(ii) optional modification of the EOI; and
(iii) delivery of the optionally modified EOI from the cell body of the sensory neuron to the spinal cord via the central branch of the sensory neuron.

13. A screening method for identification and/or validation of an EOI useful in the prevention and/or treatment of pain which comprises the following steps:
(i) administration of a lentiviral vector system as defined in any preceding claim to a non-human subject such that it delivers a test EOI to a target cell in the subject by the method as described in any of claims 1 to 11, wherein the target cell is in *situ* within the DRG;
(ii) analysis of effect of the EOI on the target cell; and
(iii) selection of an EOI with therapeutic potential.

14. A method according to claim 13, wherein the step (ii) comprises monitoring EOI-induced modulation of the transcriptome and/or proteosome of the target cell.

15. A method according to claim 13, which involves analysis of pain in the subject.

16. A method according to claim 15, wherein the perception and/or transmission of pain in the subject is analysed.

17. A method according to claim 15 or 16, which comprises the following steps:
(vi) *in vitro* selection of an EOI with therapeutic potential;
(vii) delivery of the selected EOI to the DRG of the subject; and
(iii) analysis of pain in the subject.

## Patentansprüche

1. Verwendung eines lentiviralen Vektorsystems, das mit wenigstens einem Teil eines Rabies G-Proteins oder einer Mutante, einer Variante, einem Homologen oder einem Fragment davon pseudotypisiert ist, bei der Herstellung eines Medikaments zur Verwendung in einem Verfahren zur Behandlung und/oder zur Unterdrückung von Schmerzen in einem Subjekt, wobei das Verfahren die Stufe des Verabreichens des lentiviralen Vektorsystems, so daß es eine interessierende Einheit (EOI) an ein Spinalganglion (DRG) des Subjekts zuführt, umfaßt, wobei
(i) das Vektorsystem oder ein Teil davon sich durch retrograden Transport von der Verabreichungsstelle zum DRG bewegt und
(ii) die Pseudotypisierungseinheit die Wirkung besitzt, daß sie ermöglicht, daß sich das Vektorsystem durch retrograden Transport zum DRG bewegt.

2. Verwendung nach Anspruch 1, wobei die Verabreichungsstelle eine periphere Stelle ist.

3. Verwendung nach Anspruch 1 oder 2, wobei das Vektorsystem mittels Injektion in den Bereich des Schmerzes an das Subjekt verabreicht wird.

4. Verwendung nach einem der vorangegangenen Ansprüche, wobei die EOI die zelluläre Erregbarkeit einer Zielzelle modulieren kann.

5. Verwendung nach Anspruch 4, wobei die EOI eine Hyperpolarisation der Zielzelle verursachen kann.

6. Verwendung nach einem der vorangegangenen Ansprüche, wobei die EOI die Expression und/oder Aktivität eines Ionenkanals modulieren kann.

7. Verwendung nach Anspruch 6, wobei die EOI die Expression eines lonenkanals oder eines Teils davon verursachen kann.

8. Verwendung nach Anspruch 7, wobei der Ionenkanal konstitutiv aktiv ist.

9. Verwendung nach einem der vorangegangenen Ansprüche, wobei in dem Vektorsystem
(iii) die EOI eine interessierende Nukleotidsequenz (NOI) ist,
(iv) die Expression der NOI der Kontrolle eines anzielbaren Promotors untersteht und
(v) der anzielbare Promotor die Expression der NOI auf C-Fasern und/oder Aδ-Fasern beschränkt.

10. Verwendung nach einem der Ansprüche 1 bis 8, wobei in dem Vektorsystem
(i) die EOI eine NOI ist und
(ii) die Expression der NOI induzierbar ist.

11. Verwendung nach einem der vorangegangenen Ansprüche, wobei die EOI an den Zellkörper eines sensorischen Neurons innerhalb des DRG zugeführt wird.

12. Verwendung nach Anspruch 11 für die Zuführung einer EOI in das Rückenmark, wobei das Verfahren die folgenden Stufen umfaßt:
(i) Zuführen einer EOI an den Zellkörper eines sensorischen Neurons innerhalb des DRG,
(ii) optional Modifizieren der EOI und
(iii) Zuführen der optional modifizierten EOI von dem Zellkörper des sensorischen Neurons zum Rückenmark über das zentrale Axon des sensorischen Neurons.

13. Screeningverfahren zum Identifizieren und/oder Validieren einer EOI, die bei der Unterdrückung und/oder Behandlung von Schmerzen von Nutzen ist, wobei das Verfahren die folgenden Stufen umfaßt:
(i) Verabreichen eines lentiviralen Vektorsystems wie in einem der vorangegangenen Ansprüche definiert an ein nicht-menschliches Subjekt, so daß es durch das in einem der Ansprüche 1 bis 11 beschriebene Verfahren eine Test-EOI an eine Zielzelle in dem Subjekt zuführt, wobei die Zielzelle innerhalb des DRG *in situ* vorliegt,
(ii) Analyse der Wirkung der EOI auf die Zielzelle und
(iii) Auswählen einer EOI mit therapeutischem Potential.

14. Verfahren nach Anspruch 13, wobei Stufe (ii) das Überwachen der EOI-induzierten Modulation des Transkriptoms und/oder Proteosoms der Zielzelle umfaßt.

15. Verfahren nach Anspruch 13, welches die Analyse der Schmerzen in dem Subjekt umfaßt.

16. Verfahren nach Anspruch 15, wobei die Wahrnehmung und/oder Übertragung von Schmerzen in dem Subjekt analysiert wird.

17. Verfahren nach Anspruch 15 oder 16, welches die folgenden Stufen umfaßt:
(vi) *in vitro*-Auswahl einer EOI mit therapeutischem Potential,
(vii) Zuführen der ausgewählten EOI an das DRG des Subjekts und
(viii) Analyse der Schmerzen in dem Subjekt.

## Revendications

1. Utilisation d'un système de vecteur lentiviral pseudotypé avec au moins une partie de la protéine G rabique ou d'un de ses mutants, variants, homologues ou fragments, dans la production d'un médicament destiné à être utilisé dans une méthode pour le traitement et/ou la prévention de la douleur chez un sujet, méthode qui comprend l'étape d'administration du système de vecteur lentiviral de telle sorte qu'il délivre une entité intéressante (EOI) au ganglion de la racine dorsale (DRG) du sujet, dans laquelle
(i) le système de vecteur ou une partie de celui-ci passe du site d'administration au DRG par transport rétrograde ; et
(ii) ladite entité de pseudotypage a pour activité de permettre au système de vecteur de passer par transport rétrograde au DRG.

2. Utilisation suivant la revendication 1, dans laquelle le site d'administration est un site périphérique.

3. Utilisation suivant la revendication 1 ou 2, dans laquelle le système de vecteur est administré au sujet par injection dans la zone de la douleur.

4. Utilisation suivant l'une quelconque des revendications précédentes, dans laquelle la EOI est capable de moduler l'excitabilité cellulaire d'une cellule cible.

5. Utilisation suivant la revendication 4, dans laquelle la EOI est capable de provoquer une hyperpolarisation de la cellule cible.

6. Utilisation suivant l'une quelconque des revendications précédentes, dans laquelle la EOI est capable de moduler l'expression et/ou l'activité d'un canal ionique.

7. Utilisation suivant la revendication 6, dans laquelle la EOI est capable de provoquer l'expression d'un canal ionique ou d'une partie de celui-ci.

8. Utilisation suivant la revendication 7, dans laquelle le canal ionique est constitutivement actif.

9. Utilisation suivant l'une quelconque des revendications précédentes, d'un système de vecteur dans lequel :
(iii) la EOI est une séquence de nucléotides intéressante (NOI)
(iv) l'expression de la NOI est sous le contrôle d'un promoteur ciblé ; et
(v) le promoteur ciblé restreint l'expression de la NOI aux fibres C et/ou aux fibres Aδ.

10. Utilisation suivant l'une quelconque des revendications 1 à 8, d'un système de vecteur dans lequel :
(i) la EOI est une NOI ; et
(ii) l'expression de la NOI est inductible.

11. Utilisation suivant l'une quelconque des revendications précédentes, dans laquelle la EOI est délivrée au corps cellulaire d'un neurone sensoriel dans le DRG.

12. Utilisation suivant la revendication 11, pour délivrer une EOI à la moelle épinière, qui comprend les étapes suivantes :
(i) administration d'une EOI au corps cellulaire d'un neurone sensoriel dans le DRG ;
(ii) modification éventuelle de la EOI ; et
(iii) administration de la EOI éventuellement modifiée du corps cellulaire du neurone sensoriel à la moelle épinière par la branche centrale du neurone sensoriel.

13. Méthode de dépistage pour l'identification et/ou la validation d'une EOI utile dans la prévention et/ou le traitement de la douleur, qui comprend les étapes suivantes :
(i) administration d'un système de vecteur lentiviral tel que défini dans l'une quelconque des revendications précédentes à un sujet non humain de telle sorte qu'il délivre une EOI d'essai à une cellule cible chez le sujet par la méthode telle que décrite dans l'une quelconque des revendications 1 à 11, où la cellule cible est présente in situ dans le DRG ;
(ii) analyse de l'effet de la EOI sur la cellule cible ; et
(iii) sélection d'une EOI ayant un potentiel thérapeutique.

14. Méthode suivant la revendication 13, dans laquelle l'étape (ii) comprend le contrôle de la modulation, induite par la EOI, du transcriptome et/ou du protéosome de la cellule cible.

15. Méthode suivant la revendication 13, qui comprend l'analyse de la douleur chez le sujet.

16. Méthode suivant la revendication 15, dans laquelle la perception et/ou la transmission de la douleur chez le sujet sont analysées.

17. Méthode suivant la revendication 15 ou 16, qui comprend les étapes suivantes :
(vi) sélection *in vitro* d'une EOI ayant un potentiel thérapeutique ;
(vii) administration de la EOI choisie au DRG du sujet ; et
(iii) analyse de la douleur chez le sujet.
